(19) Europäisches Patentamt
European Patent Office
Office européen des brevets

(11) **EP 2 621 550 B1**

(12) **EUROPÄISCHE PATENTSCHRIFT**

(45) Veröffentlichungstag und Bekanntmachung des Hinweises auf die Patenterteilung:
**30.08.2017 Patentblatt 2017/35**

(21) Anmeldenummer: **11787588.0**

(22) Anmeldetag: **29.09.2011**

(51) Int Cl.:
*A61M 1/16* *(2006.01)*

(86) Internationale Anmeldenummer:
**PCT/DE2011/001785**

(87) Internationale Veröffentlichungsnummer:
**WO 2012/062257 (18.05.2012 Gazette 2012/20)**

(54) **DIALYSAT-PROFILING GESTEUERT DURCH UV-KONTROLLE**

DIALYSATE PROFILING CONTROLLED BY UV MONITORING

PROFILAGE DE DIALYSAT COMMANDÉ PAR CONTRÔLE UV

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.09.2010 DE 102010047215**

(43) Veröffentlichungstag der Anmeldung:
**07.08.2013 Patentblatt 2013/32**

(73) Patentinhaber: **B. Braun Avitum AG**
**34212 Melsungen (DE)**

(72) Erfinder:
• **WOLFF, Henrik**
**37213 Witzenhausen (DE)**
• **MOLL, Stefan**
**22587 Hamburg (DE)**

(74) Vertreter: **Winter, Brandl, Fürniss, Hübner, Röss, Kaiser, Polte - Partnerschaft mbB Patent- und Rechtsanwaltskanzlei Alois-Steinecker-Strasse 22 85354 Freising (DE)**

(56) Entgegenhaltungen:
EP-A1- 1 543 852     EP-A1- 2 005 982
EP-A1- 2 163 272     EP-A1- 2 218 472
EP-B1- 1 083 948     WO-A1-98/19592

**Beschreibung**

Die Erfindung bezieht sich auf eine Methode und eine Vorrichtung zur Bestimmung von entfernten harnpflichtigen Substanzen aus einem extrakorporalen Blutkreislauf und einer anhand dieser Daten gesteuerten Flussanpassung. Die erfindungsgemäßen Verfahren dienen zur Optimierung des Verbrauchs sowie des Einsatzes eines Volumens an Dialyseflüssigkeit.

Bei Patienten mit teilweiser oder vollständiger Niereninsuffizienz werden Abfallprodukte des natürlichen Stoffwechsels einschließlich urämischer Toxine durch Blutbehandlungsverfahren wie beispielsweise die Hämodialyse entfernt, wobei die Entfernung der Stoffe aus dem Blut extrakorporal durch den Kontakt des Blutes mit einer Dialyselösung im sog. extrakorporalen Blutkreislauf erfolgt. Der Stofftransport vom Blut in die Dialyselösung erfolgt über diffusive und konvektive Effekte. Ziel ist, daß vornehmlich urämische Toxine entfernt werden. Dies geschieht, indem für den Patienten lebensnotwendige Substanzen in physiologischer Konzentration der Dialyselösung beigefügt werden.

Das Maß für die Dialysedosis eines Patienten kann nicht nur auf Basis der zumeist subjektiven Einschätzung der Gesundheit des Patienten erfolgen. Es ist notwendig, den Dialyseerfolg in einer Weise zu quantifizieren, so daß eine ausreichende Dialyseleistung sichergestellt wird. Gleichzeitig ist ein zu hohes Maß an Dialyse aus Kostengründen ebenfalls zu vermeiden. Um die Dialysetherapie effizienter zu gestalten, ist es notwendig, die Dialyseeffizienz während der Behandlung zu kontrollieren, um diese durch Anpassung der variablen Parameter der Blutbehandlungseinheit manuell oder automatisch steuern zu können.

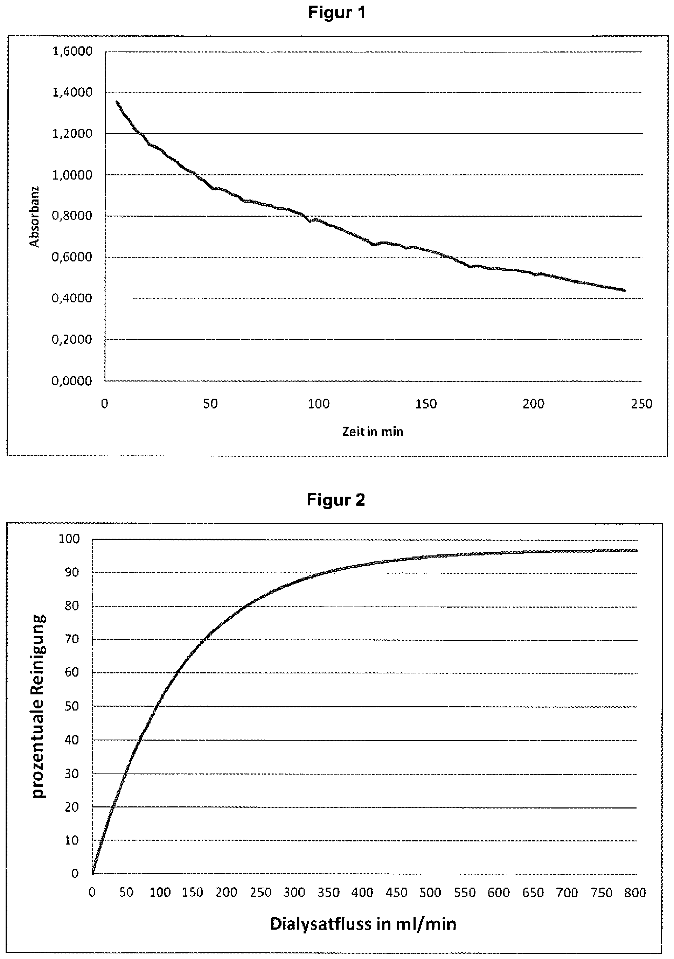

Um eine adäquate Dialysetherapie zu gewährleisten, wurde das $Kt/V$-Model entwickelt. Harnstoff (Urea) stellt das wichtigste Stoffwechselendprodukt im zu reinigenden Blut dar. Daher wird Harnstoff zur Bestimmung einer adäquaten Dialysetherapie herangezogen. K ist die Reinigungsleistung (Clearance) des Dialysators von Harnstoff aus dem Blut in ml/min, t die Behandlungszeit in min und V das Verteilungsvolumen von Harnstoff in ml im menschlichen Körper, das in direkter Relation zum Gewicht des Patienten steht. Der dimensionslose Faktor $Kt/V$ bildet ein Maß für die Reduktion von in Harnstoff gebundenem Stickstoff im Blut eines Patienten. Der zeitliche Verlauf ist in Figur 1 skizziert.

Die Bestimmung der Harnstoffkonzentration und/oder der Konzentration von anderen toxischen Substanzen im Dialysatabfluss bietet eine umfassende Überwachung des Dialyseverlaufs. Allerdings müssen dazu bis heute Proben manuell aus dem extrakorporalen System entnommen und zur chemischen Analyse an ein entsprechend ausgerüstetes Labor weitergereicht werden. Damit ist eine unmittelbare Steuerung des Dialysegerätes noch während der Dialyse des jeweiligen Patienten ausgeschlossen. Da dialysepflichtige Patienten meist chronisch erkrankt sind und sich regelmäßig einer Dialyse unterziehen müssen, fallen bei einem solchen Kontrollverfahren ständig ein erhöhter Personalbedarf und Analysekosten an. Zudem birgt die häufige Probeentnahme aus dem extrakorporalen Blutkreislauf ein kleines, aber reales Risiko der Kontamination mit Bakterien und Viren.

Daher wurde eine kontinuierliche Überwachung der Hämodialyse gefordert *(IEEE Engineering in Medicine & Biology Society 11th International Conference; Proceedings).* Ein aus dem Stand der Technik bekannter Parameter ist die Änderung der Leitfähigkeit der Dialyselösung durch die Hydrolyse von Harnstoff und/oder anderer wichtiger Moleküle. Die Kalibrierung von Leitfähigkeitssensoren, welche speziell für diese Applikation entwickelt wurden, hat sich in der Praxis als sehr mühselig und instabil erwiesen, da Einflüsse auf die Leitfähigkeit auch aus anderen Quellen stammen können. Zudem können mittels Leitfähigkeitsmessungen keine Einzelsubstanzen sondern nur die Gesamtheit der Elektrolyte gemessen werden, was recht ungenau ist.

Des Weiteren kann die kontinuierliche Überwachung einer Hämodialyse über optische Absorbanzmessung erfolgen. Die Transmission der Dialyselösung wird Wesentlichen von Harnsäure und anderen niedermolekularen Stoffen beeinflusst. Eine solche Messeinrichtung wird durch das *UV-Monitoring System* nach Fridolin in EP 1 083 948 B1 beschrieben.

In der EP 1 543 852 B1 wird ein Dialysegerät beschrieben, bei denen die Messung des Hämokrit im Blut des Patienten dazu verwendet wird, eine Idealkurve der Blut- und Behandlungsparameter aufgrund dieser Messung zu korrigieren. Eine Messung der UV-Absorbanz ist bei diesem Verfahren jedoch nicht vorgesehen. EP 2 163 272 offenbart ein Verfahren zur Vorhersage eines Dialyseleistungsparameters (z.B. Kt/V), wobei im Dialysatausfluss durch Messung der UV-Absorbanz die Konzentration einer harnpflichtigen Substanz bestimmt wird. Bei Abweichungen zwischen der gemessenen Reinigungsleistung und einer zu erreichenden Reinigungsleistung kann der Blutfluss oder der Dialysatfluss angepasst werden. Der Quotient der Produktion von Harnsäure zu Harnstoff ist weitgehend konstant in Patienten, unabhängig von dem Grad der Niereninsuffizienz. In anderen Worten, die pro Zeiteinheit gebildeten Mengen an Harnstoff und Harnsäure korrelieren recht gut miteinander. Die Eliminationsrate der beiden Substanzen ist unter physiologischen Bedingungen recht ähnlich. Daher bildet die Konzentrationsmessung der Harnsäure im Dialysatausfluss in Verbindung mit dem Fluss der Dialyselösung unmittelbar ein Maß für die Menge an entferntem Harnstoff. Der Vorteil, die Absorbanz von Harnsäure zu messen, liegt darin, daß Harnsäure im Gegensatz zu Harnstoff eine scharfe und charakteristische Bande im UV-Bereich aufweist, die zwischen 280 nm und 290 nm liegt.

Ziel der Erfindung ist es, ein online-Monitoring-System zur Verfügung zu stellen, mit dessen Hilfe der Fluss der Dialyselösung und/oder des Blutes gesteuert werden kann. Bisher ist eine solch direktes Verfahren zur Optimierung des Dialysevorgangs und seine Implementierung in einer Blutbehandlungseinheit nicht möglich, da die Daten über die Dialysequalität - wenn überhaupt erhoben - erst nach der Behandlung ermittelt werden und somit eine akute Anpassung nicht möglich ist.

Überraschenderweise wurde gefunden, daß über ein photometrisches Konzentrationsmesssystem gewonnene Daten nicht nur zur Analyse und zur Erfolgskontrolle des Dialysevorgangs verwendet werden können, sondern auch zur Berechnung der Regelung der Blutbehandlungseinheit genutzt werden können. In der vorliegenden Erfindung wird ein Verfahren und eine Vorrichtung beschrieben, bei dem die online gewonnenen Messwerte zur Steuerung des gesamten Dialysevorgangs verwendet werden können. Dieses Ziel wird durch die Bereitstellung von Verfahren und Vorrichtungen gemäß der unabhängigen Ansprüche gelöst. Weitere vorteilhafte Ausführungsformen, Aspekte und Details der Erfindung ergeben sich aus den abhängigen Ansprüchen, den Figuren und der Beschreibung.

**Beschreibung**

Grundlage dieser Erfindung ist ein online-Monitor der Dialysequalität, der mittels photometrischer Absorbanz im Dialysatausfluss die Dialyseleistung in Form des Kt/V-Wertes ermittelt. In bevorzugten Ausführungsformen wird die UV-Absorbanz gemessen.

In weiteren Ausführungsformen kann die photometrische Absorbanz alternativ oder zusätzlich im Blutfluss gemessen werden. In bevorzugten Ausführungsformen erfolgt diese blutseitige Messung zwischen Patientenzugang und Dialysatoreingang. Somit sind die folgenden Beschreibungen und Ausführungen sowohl auf dialysatseitige als auch blutseitige photometrische Absorbanz zu beziehen.

Unter dem Oberbegriff Blutbehandlungseinheit werden alle Vorrichtungen verstanden, die zur Reinigung und/oder Behandlung von Blut eingesetzt werden können. Die am häufigsten eingesetzten Verfahren sind die Hämodialyse über zwei Nadeln, Hämodialyse über eine Nadel, Cross-Over-Hämodialyse mit einer Nadel, Peritonealdialyse, Hämoperfusion, Hämodiafiltration mit Postdilution, Hämodiafiltration mit Prädilution, Hämodiafiltration mit Prä-Post-Dilution, Hämofiltration mit Postdilution, Hämofiltration mit Prädilution, Hämofiltration mit Prä-Post-Dilution oder einer sequentiellen Hämodialyse.

Der Begriff Dialyse bezieht sich auf Reinigungsverfahren, bei denen zwei Flüssigkeitsströme durch eine permeable Membran voneinander getrennt sind, die aber den gewünschten Stoffaustausch ermöglicht. Der eine Flüssigkeitsstrom, im vorliegenden Fall Blut, führt die zu entfernenden Stoffe mit sich, während der andere Strom mit der Dialyselösung diese Stoffe aufnehmen soll.

Ein wichtiges und bekanntes Dialyseverfahren ist die Hämodialyse, die zur Blutwäsche bei teilweisem oder vollständigem Nierenversagen standardmäßig durchgeführt wird. Hierbei wird extrakorporal das zu reinigende Blut über eine semipermeable Membran gegen die Dialyselösung dialysiert. Dem Blut werden harnpflichtige Schlackestoffe entzogen und das aufgereinigte Blut wird dem körpereigenen Blutkreislauf wieder zugeführt. Um zu vermeiden, daß durch die Dialyse dem Blut physiologisch wichtige Stoffe entzogen werden, wird die Dialyselösung mit eben diesen Stoffen angereichert. Damit wird eine Fokussierung auf die Entfernung der Schlackestoffe und urämischer Toxine erreicht.

Das zugrundeliegende Prinzip der Dialyse ist die Filtration, insbesondere die Tangentialflussfiltration (TFF; auch Querstromfiltration oder Cross-Flow-Filtration genannt). Das zu reinigende Blut wird durch ein Filtermodul aus Hohlfasern geleitet, den eigentlichen Dialysator oder Dialysefilter. Die Wand dieser Hohlfasern ist durch eine semipermeable Membran von der Reinigungslösung (Dialyselösung) getrennt. Die Dialyselösung weist für gewöhnlich eine niedrigere Konzentration derjenigen Stoffe auf, die aus der zu reinigenden Flüssigkeit entfernt werden sollen. Durch diesen Konzentrationsunterschied kommt es zu einer Diffusion. Um die Diffusion als Trennkraft optimal nutzen zu können, werden Tangentialflussfilter heutzutage bevorzugt im Gegenstromprinzip betrieben.

Ein weiterer Reinigungsmechanismus ist die Konvektion. Hier wird ein Druckgradient über den Dialysefilter erzeugt, wodurch die zu reinigende Flüssigkeit verstärkt über die semipermeable Membran gedrückt wird. Dadurch werden die Substanzen in ihrer vorliegenden Konzentration mit gespült. Bei der Hämodialyse ist dieser Effekt vergleichsweise gering, da dem Patienten nur das physiologisch notwendige Flüssigkeitsvolumen entzogen wird. Im Falle konvektiver Therapieformen wie Hämofiltration und Hämodiafiltration wird dem Blut über diesen Mechanismus konsequent Flüssigkeit entzogen, man spricht von Ultrafiltration. Dieser Reinigungsprozess ist daher nicht von einem Konzentrationsgradienten der betreffenden Substanzen abhängig. Entscheidend sind hier Membran- und Stoffeigenschaften wie Porendurchmesser, Filtrationsstrecke, Siebkoeffizient, Permeabilität usw. der Siebkoeffizient ist eine Funktion der Molekülgröße, der elektrischen Ladung, der Form und des Aggregatszustands der zu entfernenden Substanz. Die Permeabilität ist der Quotient aus transportierter Stoffmenge pro Zeit und dem Produkt aus dem Konzentrationsgradienten und der Durchtrittsfläche.

Das durch Ultrafiltration entfernte Flüssigkeitsvolumen sollte bis auf eine zu eliminierende natürliche Wassermenge, die der natürlichen Harnvolumen entspricht, substituiert werden. Dies kann durch Substitution mit einer Substituatlösung, in der Regel physiologischer Kochsalzlösung geschehen.

Neben den Dialysetherapieformen gibt es weitere Blutreinigungsverfahren wie die Apherese, die unabhängig von Nierenversagen unter akuten Symptomen wie Hyperkaliämie, einer metabolischen Azidose, Überwässerung (z.B. bei einem Lungenödem), urämischer Serositis (z.B. bei Perikarditis oder urämischer Enzephalopathie) oder Vergiftungen mit dialysierbaren Substanzen wie Lithium oder Acetylsalicylsäure leiden. Chronisch auftretende Symptome, die eine Dialyse indizieren, sind u.a. eine niedrige glomeruläre Filtrationsrate, Hyperphosphatämie oder Urämie. Das erfindungsgemäße Verfahren zur Steuerung von verwandten Behandlungsverfahren kann auch zur Behandlung der vorgenannten Symptome oder Erkrankungen eingesetzt werden. Die im weiteren dargestellten Blutreinigungsverfahren werden oft ebenfalls unter dem Überbegriff Dialyse subsumiert.

Ein Verfahren zur ständigen Behandlung niereninsuffizienter Patienten ist die Peritonealdialyse. Hierbei handelt es sich um ein intrakorporales Verfahren, bei dem die Membraneigenschaften des Bauchfells ausgenutzt werden. Die Bauchhöhle des Patienten wird hierbei mit der Dialysierlösung gefüllt, die über zwei Ports zu- und abgeleitet wird.

Bei der Hämoperfusion (beispielsweise bei Vergiftungen) wird das Blut extrakorporal über ein Adsorbens wie Aktivkohle oder ein Austauscherharz geführt, um die betreffenden Toxine aus dem Blut zu entfernen.

Bei der Hämofiltration wird dem Blut konvektiv Wasser entzogen. Hierbei drückt ein angelegter Druckgradient das Blut über eine Membran. Damit werden auch harnpflichtige Substanzen mit ausgespült. Der Volumenverlust wird durch Zugabe von Elektrolytlösung kompensiert.

Bei dem Verfahren der Hämodiafiltration werden die klassische Hämodialyse und die Hämofiltration miteinander kombiniert, um sich die Vorteile beider Verfahren zu Nutze zu machen. Auf diese Weise können Moleküle mit niedrigem und mittlerem Molekulargewicht eliminiert werden. Daher stellt die Hämodiafiltration ein bevorzugtes Blutreinigungsverfahren dar, bei dem die vorliegende Erfindung angewendet werden kann.

Der Begriff Ultrafiltration bezieht sich hierbei auf die Filtration über eine Membran mit einem Druck von 0 - 1 bar, bei der Teilchen größer als ca. 0,01 $\mu$m zurückgehalten werden sollen. Dies entspricht der Größe mittelmoleklarer Substanzen, aber auch von Makromolekülen, Viren und Kolloiden.

Mit den demographischen Veränderungen und den damit verbundenen Veränderungen in der Alterspyramide einer Gesellschaft gewinnen diese Verfahren v.a. in den Industriestaaten eine stetig steigende Bedeutung, da die Anzahl chronisch niereninsuffizienter Patienten kontinuierlich anwächst. So gibt es zur Zeit beispielsweise in Deutschland ca. 60.000 dialysepflichtige Patienten pro Jahr. Die Sterblichkeit liegt aber noch bei ca. 20% pro Jahr. Die Inzidenz von Neuerkrankungen beträgt 184 pro 1 Million Einwohner. Auch in den aufstrebenden Wirtschaftsmächten wird die Zahl zu behandelnder Patienten in den nächsten Jahren stark ansteigen.

Eine durchschnittliche Hämodialysebehandlung dauert 4 bis 5 Stunden. Eine Nachtdialyse dauert bis zu 8 Stunden. Eine solche Behandlung ist bei den meisten Patienten mindestens dreimal in der Woche nötig. Die Behandlungshäufigkeit hängt u.a. vom Körpergewicht, der Nierenrestfunktion und der Herzleistung des Patienten ab. Die Tendenz geht jedoch dahin, eher mehrere kürze Behandlungen durchzuführen.

Um diesen immensen Bedarf zu decken, bedarf es einer aufwändigen Infrastruktur, und damit verbunden, eines großen Personalbedarfs. Auch der Materialverbrauch ist gewaltig. So passieren bei einer durchschnittlichen 5-stündigen Dialyse ca. 150 Liter Dialyselösung den Dialysator. Hochgerechnet bedeutet dies, daß ein Dialysepatient pro Jahr ca. 23.000 Liter Dialyselösung benötigt. Da aufgrund der hohen Qualitätsansprüche an Dialyselösung ein Beutel trotz der chemisch einfachen Zusammensetzung teuer ist, belaufen sich die Behandlungskosten eines Dialysepatienten pro Jahr auf ca. 43.000 €. Bei 60.000 Patienten wären dies allein für Deutschland ca. 2,58 Milliarden €. Dies ist ein beträchtlicher Bestandteil des öffentlichen Gesundheitsbudgets. Daher ist es von allgemeinem Interesse, Einsparpotentiale bei Dialysebehandlungen möglichst umfangreich auszuschöpfen.

Bei diesen immensen Kosten erscheint es geboten, Einsparungsmöglichkeiten zu realisieren, wobei die Qualität der medizinischen Behandlung jedoch nicht leiden darf. Das bedeutet, daß die harnpflichtigen Substanzen weiterhin in einem medizinisch vertretbaren Prozentsatz aus dem Blut des Patienten entfernt werden müssen. Um dies sicher zu stellen, muß die Konzentration der zu entfernenden Leitsubstanzen zuverlässig ermittelt werden.

Mit dem erfindungsgemäßen Verfahren wird eine Optimierung der Dialyseeffizienz angestrebt. Die Dialyseeffizienz ergibt sich aus dem Zusammenspiel von den bei der Dialyse eingesetzten Kosten und der Dialyseleistung. Für die Dialyseleistung muß ein eigenes Maß gefunden werden. In der wissenschaftlichen Literatur haben sich eine Reihe von Berechnungen etabliert, um die Dialyseleistung auszudrücken. Einige der Werte sind sehr ähnlich, während andere einen unterschiedlichen Schwerpunkt bei der Betrachtung des Problems setzen. Erfindungsgemäß ist das Verfahren in der Lage, Messwerte für alle etablierten Kenngrößen und Kombinationen davon zur Verfügung stellen zu können, damit diese bei der Steuerung des Dialysevorgangs berücksichtigt werden können.

Die am häufigsten verwendete Größe für einen Dialyseleistungsparameter ist das sog. Kt/V-Modell. Harnstoff (Urea) ist das wichtigste metabolische Endprodukt im zu reinigenden Blut. Daher ist die Harnstoffkonzentration ein hervorragender Parameter, anhand dessen die Leistung einer adäquaten Dialysetherapie nachvollzogen werden kann. K ist die Reinigungsleistung (Clearance) des Dialysators von Harnstoff aus dem Blut in ml/min, t die Behandlungszeit in min und V das Verteilungsvolumen von Harnstoff in ml im menschlichen Körper, das direkt in Zusammenhang mit dem Patientengewicht steht. Der dimensionslose Faktor Kt/V bildet die Reduktion von Harnstoff-Stickstoff im Blut eines Patienten ab.

Bei, der Hämodialyse werden Kt/V-Werte $\geq 1,2$ angestrebt. Bei einem technisch problemlosen Verlauf einer Dialyse kann ein solcher Wert standardmäßig erreicht werden.

Nach Daugirdas ist

$$K \cdot \frac{t}{V} = -\ln\left[\frac{C_t}{C_0} - 0,008 \cdot t + \left(4 - 3,5\frac{C_t}{C_0}\right) \cdot \frac{UF}{Gew}\right]$$

Hierbei bedeutet;

$C_i$ Hamstoffkonzentration am Ende der Dialyse
$C_0$ Harnstoftkonzentration vor Beginn der Dialyse
t Dialysezeit in Stunden
UF Ultrafiltrationsvolume in Liter
K Clearance in ml/min
Gew Trockengewicht in kg.

(zitiert in: Halwachs-Baumann: Labormedizin: Klinik - Praxis -Fallbeispiele. 2. Aufl. Springer Wien New York, 2011, S. 298).

Eine einfache Größe ist die URR (urea reduction ratio):

$$URR\ [\%] = (C_0 - C_t) / C_0 * 100$$

$C_0$ ist die Konzentration von Harnstoff zum Zeitpunkt 0 (Beginn der Dialyse oder des Behandlungszyklus). $C_0$ ist die Konzentration von Harnstoff zum aktuellen Zeitpunkt t. URR [%] ist der Prozentsatz, der zum Zeitpunkt t eliminiert wurde. Es werden Werte größer als 65% angestrebt.

spKt/V (single-pool Kt/V) berücksichtigt sowohl die Harnstoffproduktion während der Dialyse als auch den Effekt der Ultrafiltration:

$$spKt/V = -\ln(R - 0,008 * t) + (4 - 3,5 * R) * UF / W$$

Hierbei ist $R = C_t / C_0$, t die Dialysedauer in Stunden, UF das Ultrafiltrationsvolumen in kg und W das Gewicht nach der Hämodialyse in kg.

Der eKtN - Wert (equilibrated Kt/V) berücksichtigt zudem den Harnstoffrebound, der noch nach Dialyseende stattfindet. Der Harnstoffrebound bezeichnet den Effekt, daß nach Ende der Dialyse die Harnstoffkonzentration im Blut vergleichsweise schnell wieder ansteigt, da nun der in gering durchbluteten Geweben vorhandene und durch die Dialyse nicht erfassbare Harnstoff verstärkt in den Gesamtorganismus übertritt.

$$eKt/V = spKt/V - 0,6 / t \cdot spKt/V + 0.03$$

Diese Formel gilt für eine Dialyse über einen peripheren Shunt.

TAC/TAD ist ein Maß, wie gleichmäßig sich die Harnstoffkonzentration im Blut über einen Gesamtzeitraum darstellt. Dies ist vor allem in der Hinsicht interessant zu beurteilen, ob das gewählte Dialyseregime und die damit verbundene Dialysedosis den gewünschten Erfolg zeitigt. Hierbei bezeichnet TAC die mittlere wöchentliche Harnstoffkonzentration (time average concentration). TAD bezieht sich auf die Fluktuation der TAC-Werte, ist also eine abgeleitete Größe. Es werden geringe TAD-Werte angestrebt, da dies ein Zeichen ist, das gefährliche und potentiell toxische Spitzenwerte in der Harnstoffkonzentration selten oder überhaupt nicht auftreten.

EKR beschreibt die Harnstoff-Clearance. Hierbei ist

$$EKR = G / TAC.$$

G steht hier für die Bildungsrate von Harnstoff.

RU bezieht sich auf die Menge des eliminierten Harnstoffs (removed urea).

SRI hingegen steht für solute removal index und betrachtet die Dialyseleistung von der umgekehrten Seite:

$$\mathrm{SRI} = 1 - (V_{post} \star C_{post}) / (V_{prä} \star C_{prä})$$

($V_{post}$: Volumen nach der Dialyse; $C_{post}$: Konzentration nach der Dialyse;
$V_{prä}$: Volumen vor der Dialyse; $C_{prä}$: Konzentration vor der Dialyse)

V bezeichnet das Verteilungsvolumen von Harnstoff in Blut (siehe oben: Kt/V).

K ist ein Maß für die Clearance-Leistung eines bestimmten Dialysators. Es entspricht damit der effective body clearance.

All diese zuvor beschriebenen Kenngrößen lassen sich im Sinne der Erfindung unter dem Begriff "Dialyseleistungsparameter" zusammenfassen.

Erfindungsgemäß können in analoger Weise Messwerte für die Reinigung weiterer wichtiger harnpflichtiger Substanzen erhoben werden. Daher gelten die in der Beschreibung getätigten Darstellungen und Ausführungsbeispiele gleichermaßen für diese Substanzen. Neben Harnstoff und Harnsäure können dies u.a. Kreatinin und Hippursäure sein.

Die erfindungsgemäß erhobenen Messwerte können mit einem zuvor angelegten Profil für dieses spezielle Gerät und/oder Patienten abgerufen werden. Dieses Profil kann beispielsweise benötigte Ausgangswerte bereitstellen. Dadurch kann die Behandlung schon frühzeitig mit Geräteeinstellungen nahe der optimalen Dialyseeffizienz beginnen.

Für eine optimierte Reinigungsleistung ist es speziell im Fall des diffusiven Stofftransports von großer Bedeutung, den Fluss der Dialyselösung an die Behandlungsbedingungen anzupassen oder einen Hinweis zu erhalten, wie die Behandlung in wirtschaftlicher Hinsicht optimiert werden kann. Der Zusammenhang zwischen Dialysatfluss und Reinigungsleistung ist in Figur 2 skizziert. Die Optimierung erfolgt vorwiegend nach ökonomischen Kriterien. Therapeutische Gesichtspunkte können gegebenenfalls auch berücksichtigt werden. Diese können in die Vorauswahl des Operationsmodus und eines untersten akzeptablen Werts für einen Dialyseleistungsparameters eingehen, haben dann aber keinen Einfluss mehr auf die Optimierung nach ökonomischen Kriterien.

Eine Ökonomisierung, d.h. ein Einsparen der bei den benötigten Mengen kostenintensiven Dialyselösung, kann dann erzielt werden, wenn erkannt wird, daß eine Erniedrigung des Flusses der Dialyselösung keine Verringerung der Reinigungsleistung zur Folge hat. Dies kann der Fall sein, wenn beispielsweise ein großflächiger Filter mit einem hohen Dialysatfluss gekoppelt wird. In diesem Fall kann es vorkommen, daß das Blut nach beispielsweise dem halben Weg durch den Filter bereits eine Konzentration an urämischen Toxinen aufweist, die gegen sehr kleine Werte tendiert. In diesem Fall kann der Fluss der Dialyselösung reduziert werden, ohne eine Verschlechterung der Reinigungsleistung zu verursachen. Das einzige, was sich verändert, ist, daß das Blut nicht schon auf halber Strecke im Filter weitgehend vollständig gereinigt ist, sondern erst gegen Ende der Filtrationsstrecke. Man kann daher die Behandlung dahingehend optimieren, daß das Potential des Filters vollständig ausgeschöpft wird, um so den Verbrauch an Dialyselösung so gering wie möglich zu halten.

Es sei daher ausdrücklich darauf hingewiesen, daß es sich bei dem erfindungsgemäßen Verfahren ausschließlich um ein Verfahren zur Optimierung des Verbrauchs an Dialyseflüssigkeit handelt. Die basierend auf diesem Verfahren vorgenommenen Änderungen der Einstellung an der Dialysemaschine haben keinen Einfluss auf die Diagnose des Patienten, auf die Behandlungsart, auf die Behandlungseffizienz und/oder den Behandlungserfolg. Das erfindungsgemäße Verfahren dient lediglich wirtschaftlichen Überlegungen zum intelligenten sparsamen Einsatz der Dialysemaschine. Dies hat für den Patienten selber keinerlei positive oder negative Auswirkungen.

Bisher erfolgt keine Verwendung des ermittelten photometrischen Signals zur Steuerung des Dialyseprozesses, da die Daten über die Dialysequalität und den dazugehörigen Prozess - wenn überhaupt erhoben - erst ex *posteriori* ermittelt werden und somit eine zeitnahe Intervention nicht möglich ist. Auch von Signalen der Leitfähigkeit in der Dialyselösung ist eine solche Steuerung nicht bekannt.

Im umgekehrten Fall kann es vorkommen, daß eine Erhöhung des Dialysatflusses eine Steigerung der Reinigungsleistung bewirkt. Dies ist der Fall, wenn das Blut den Dialysator verlässt, aber noch eine relativ hohe Konzentration an urämischen Substanzen aufweist. Durch eine Erhöhung des Flusses der Dialyselösung kann so der Konzentrationsgradient im Filter erhöht und damit auch die Reinigungsleistung verbessert werden. Ziel ist es, den für die aktuelle Situation optimalen Fluss der Dialyselösung zu erzeugen.

Eine Anpassung des Flusses der Dialyselösung kann entweder in vordefinierten oder frei wählbaren Abständen erfolgen oder auf Grund von Auffälligkeiten im online-Signal der Reinigungsüberwachung. Bleibt beispielsweise das Signal über einen bestimmten Zeitraum weitgehend unverändert und/oder zeigt sehr hohe Absolutwerte an, so ist zu vermuten, daß der Filter über sein Leistungspotential hinaus beansprucht wird. Dementsprechend muß der Fluss der Dialyselösung erhöht werden.

Im umgekehrten Fall besteht bei sehr niedrigen Werten der online-Überwachung die Möglichkeit, den Fluss der Dialyselösung zu erniedrigen.

In bevorzugten Ausführungsformen wird ebenfalls die jeweils eingesparte Menge an Dialyselösung als Absolutwert und/oder bezogen auf ein Zeitintervall angezeigt.

Es besteht auch die Möglichkeit, zu Behandlungsbeginn das Volumen an Dialyselösung festzulegen, das während der Behandlung aufgewendet werden soll und die gewünschte Reinigungsleistung gewährleistet. Dieser Wert kann entweder durch einen über die Therapie konstanten Fluss erreicht werden, oder auch durch intelligent gestaffelte Profile. Im Gegensatz zu einem konstanten Fluss hat ein Profil, das mit hohen Flussraten beginnt und diese über den Behandlungsverlauf senkt, den Vorteil, dass zu Beginn der Therapie der Grundstein einer guten Dialysetherapie gelegt wird und der Qualitätsverlust gering gehalten wird, wenn gegen Ende der Behandlung Komplikationen auftreten.

Des Weiteren betrifft die Erfindung ein Verfahren, bei dem der Fluss der Dialyselösung während einer Dialysesitzung variiert wird. Über ein solches Verfahren werden Messwerte erhalten, die, wenn man sie zueinander in Bezug setzt, einen Hinweis darauf geben, ob der Fluss des Blutes und/oder der Dialyselösung nachgeregelt werden müssen oder im Sinne der Wirtschaftlichkeit nachgeregelt werden können. Für die Notwendigkeit der Nachregelung kann ein Toleranzintervall festgelegt werden, innerhalb dessen Abweichungen von einem Erwartungswert für den jeweiligen Zeitpunkt toleriert werden, bei Überschreiten des Toleranzintervalls jedoch nachgeregelt werden muß. Der Erwartungswert ergibt sich aus einer Extrapolation der ersten Messwerte während einer Dialysesitzung. Zudem können Erfahrungswerte für den jeweiligen Bautyp des Dialysators, für den individuellen Dialysator und aus der Patientenhistorie in den Erwartungswert mit einfließen. Zur Variation des Flusses der Dialyselösung werden ein Minimalwert und ein Maximalwert festgelegt, zwischen denen über den ganzen Variationsbereich der Fluss der Dialyselösung variiert wird. Dies kann nach einem vorgegebenen Schema durchgeführt werden oder durch Eingabe eines Anwenders.

Abweichungen von den Grenzen der Toleranzintervalle und/oder des interpolierten Dialyseverlaufs können zum Anlass genommen werden, eine weitere Variation des Flusses der Dialyselösung vorzunehmen.

Zudem kann ein Zeitraum festgelegt werden, innerhalb dessen die Variation durchgeführt wird. Ebenfalls kann die Änderungsgeschwindigkeit festgelegt werden, mit der die Variation ausgeführt wird. Diese Variation kann mehrere Male während einer Dialysesitzung durchgeführt werden. Auch diese Variation kann in festgelegten Intervallen oder individuell nach Bedarf stattfinden. In bevorzugten Ausführungsformen sind die Zeitintervalle am Anfang einer Dialysesitzung kürzer als an deren Ende, da für gewöhnlich gegen Ende eine geringere Variabilität des Messergebnisses auftritt. Für dieses Verfahren gelten dieselben in der vorliegenden Erfindung beschriebenen Ausführungsformen und Modifikationen.

Erfindungsgemäß kann der Fluss der Dialyselösung auch bei Bedarf variiert werden. Dies ist insbesondere der Fall, wenn die photospektroskopischen Messwerte Auffälligkeiten aufweisen, die einen Hinweis auf einen unerwarteten und/oder nicht ordnungsgemäßen Verlauf der Dialysesitzung hinweisen.

Ein solcher auffälliger Verlauf kann in einem sehr langsamer Abfall oder einem sehr großen Wert der entfernten Stoffmenge bestehen. Als Maßnahme zur Abhilfe würde eine Erhöhung des Flusses der Dialyselösung vorgeschlagen oder vorgenommen.

Umgekehrt kann der auffällige Verlauf in einem sehr geringen Wert der entfernten Stoffmenge bestehen. In diesem Fall würde eine Erniedrigung des Flusses der Dialyselösung vorgeschlagen oder vorgenommen.

Tritt eine der beschriebenen Auffälligkeiten auf, kann auch im Falle des zu Behandlungsbeginn gewählten Volumens an Dialysatflüssigkeit dieses überprüft und gegebenenfalls wie oben beschrieben angepasst werden.

Zusammenfassend lässt sich die Erfindung folgendermaßen beschreiben:

Während einer Dialysebehandlung wird mittels eines online-Messverfahrens die Konzentration mindestens einer entfernten Substanzen im Dialysat überwacht und auf Grund dieses Signals der Fluss der Dialyselösung so angepasst, daß eine optimale Sättigung der Dialyselösung an urämischen Substanzen zustande kommt.

Im Falle der Bestimmung des Volumens an Dialysatflüssigkeit wird dieser Prozess zu Anfang der Therapie herangezogen, um das Volumen an Dialysatflüssigkeit zu bestimmen, das für die Therapie aufzuwenden ist.

Um den optimalen Fluss zu ermitteln, wird der Dialysatfluss variiert und die erzeugte Absorptionsänderung analysiert.

Der oben beschriebene Effekt kann auch erzeugt werden, indem der Fluss der zu reinigenden Flüssigkeit, des Blutes, angepasst wird. Entscheidend sind oftmals nicht die absoluten Flüsse der einzelnen Kompartimente, sondern der relative Fluss der beiden zueinander.

Die Abtastrate (sampling rate) für das photospektroskopische Signal kann entweder vom Hersteller voreingestellt sein oder von den Anwendern eingegeben werden. Erfindungsgemäß liegen die Abtast- und Regelintervalle zwischen 0,5 Sekunden und 30 Minuten, bevorzugt zwischen 1 Sekunde und 10 Minuten, bevorzugter zwischen 2 Sekunden und 5 Minuten und am meisten bevorzugt zwischen 10 Sekunden und 1 Minute.

In einigen Ausführungsformen können sich jedoch die Messintervalle während einer Dialysebehandlung verlängern, entweder gemäß einem vorgegebenen Algorithmus oder nach individuellen Einstellungen. Hintergrund hierfür ist, daß sich die Behandlungsparameter erfahrungsgemäß bei Beginn einer Dialysebehandlung stärker ändern als gegen Ende, an dem sich meist ein "quasi steady state" einstellt.

In weiteren Ausführungsformen werden zunächst mindestens zwei Messpunkte aufgenommen. Diese werden zur Charakterisierung der Reinigungsleistung herangezogen. Ein weiterer Kurvenverlauf wird interpoliert. Hierbei können die Messpunkte mit einer konstanten Frequenz aufgenommen werden oder die Messabstände können auf den erwarteten Verlauf hin optimiert werden.

Bevorzugt werden photospektroskopische Methoden. Hierbei fällt der Messstrahl senkrecht zur Flussrichtung der Dialyselösung ein. Ein IR-Messstrahl wird bereits bei vielen modernen Dialysemaschinen eingesetzt. Er dient zur Detektion von sog. Blutlecks in der semipermeablen Membran des Dialysemoduls. Wenn hier ein Leck auftritt, gelangt Blut ungefiltert in die Dialyselösung und wird dadurch der Rückführung in den Körper des Patienten entzogen. Je nach Ausmaß des Lecks kann dies lebensgefährlich für einen Patienten sein. Das ausgetretene Blut kann durch eine Trübung in der Dialyselösung detektiert werden und daraufhin kann die Dialysesitzung unter- oder abgebrochen werden. Das Problem beim Messen wässriger Lösungen sind jedoch zwei sehr breite charakteristische Banden für $H_2O$, die viele Messwerte überdecken. IR-Messungen sind daher nur für die Konzentrationsmessungen von Interesse, bei der die Leitsubstanz mindestens eine charakteristische Bande außerhalb der beiden $H_2O$-Bande aufweist.

Polarisiertes Licht kann auch nur bei Leitsubstanzen verwendet werden, die erstens ein chirales Zentrum haben und falls sie ein solches haben, nicht als Racemat vorliegen. Da Harnstoff nicht chiral ist, kann solch eine Methode nicht zur Harnstoffmessung verwendet werden. Bei anderen Leitsubstanzen, die diese Bedingung erfüllen, würde sich die Konzentration c wie folgt berechnen:

$$c = \alpha \, / \, (\alpha_t \cdot d)$$

($\alpha$ = gemessener Drehwinkel; $\alpha_t$ = spezifisches Drehvermögen einer Substanz; d = Messstrecke, hier der Durchmesser des Dialysatausflusses; $\alpha$ und $\alpha_t$ sind wellenlängenspezifisch).

Besonders bevorzugt wird daher die Verwendung mindestens einer UV-Absorbanz-Messeinheit. Hierbei wird ein UV-Lichtstrahl durch ein Segment der zu messenden Lösung geleitet und von einem Empfänger gemessen. Aus diesem Messwert läßt sich die Extinktion, oder als Kehrwert die Transmission, bei einer bestimmten Wellenlänge im UV-Bereich (1 - 400 nm Wellenlänge) berechnen. Die Extinktion ist für einen großen Konzentrationsbereich bei den meisten Substanzen linear zur Konzentration. Diese wird allgemein durch das Lambert-Beer-Gesetz beschrieben:

$$E_\lambda = \varepsilon_\lambda \cdot c \cdot d$$

Die Extinktion $E_\lambda$ ist das Produkt aus dem stoffspezifischen molaren Extinktionskoeffizienten $\varepsilon_\lambda$, der Stoffkonzentration c und der Wegstrecke des gemessenen Lichtes d.

Da $\varepsilon_\lambda$ und d für eine Apparatur festgelegt sind, ist $E_\lambda$ ein direktes Maß für die zu messende Stoffkonzentration.

Die UV-Messung ist stark temperaturabhängig, da die Temperatur direkt in den Extinktionskoeffizienten $\varepsilon_\lambda$ eingeht. Daher werden in bevorzugten Ausführungsformen Vorrichtungen getroffen, daß die Temperatur der Dialyselösung zumindest im Dialysatausfluß konstant gehalten wird, um eine Vergleichbarkeit der Messwerte zu gewährleisten.

Für die UV-Absorptionsmessung können handelsübliche Sensoren verwendet werden. In bevorzugten Modellen fließt der Flüssigkeitsstrom durch ein transparentes Ausflussteil, beispielsweise einen Schlauch, der durch eine zentrale Aussparung am UV-Sensor geführt wird. In anderen Worten, der UV-Sensor umgibt das Ausflussteil in einem Abschnitt ringförmig. Der UV-Strahl fällt hierbei durch das zentrale Segment des Ausflussteiles in seiner ganzen Breite. Dies führt zu einer Erhöhung der Messgenauigkeit, da Streuungs- und Beugungseffekte so reduziert werden. Bei der Auswahl des zumindest in diesem Abschnitt transparenten Wandmaterials des Ausflussteiles ist es vorteilhaft, wenn Materialien verwendet werden, bei denen nur eine geringe Lichtstreuung und Reflexion auftritt. Diese beiden Effekte wirken sich ebenfalls negativ auf die Messgenauigkeit aus. Insbesondere bei Messungen unterhalb von 200 nm Wellenlänge hat sich Quarzglas bewährt. Erfindungsgemäß kann dieses Glas auch in den Sensor eingebaut sein und auf der Wegstrecke durch den Sensor fließt die Dialyselösung frei durch dieses Glasrohr. Entsprechende Anschlüsse vor und nach dem Sensor herzustellen, liegt im Wissensbereich des durchschnittlichen Fachmanns auf diesem Gebiet. Die Fehlergenauigkeit des Messsystems sollte bevorzugt unter 15% liegen, bevorzugter unter 10% und am meisten bevorzugt unter 5%.

Aus Schutzgründen sollten, wenn aufgrund der Bauweise nötig, Sichtblenden um den Messbereich des UV-Sensors angebracht sein, um zu verhindern, daß menschliches Gewebe, vor allem aber die Augen des Personals und/oder der Patienten, mit dem UV-Messstrahl in Berührung kommt.

Die Konzentrationsmessung der zu entfernenden Substanz oder Substanzen kann erfindungsgemäß an allen Stellen in dem Dialysekompartiment vorgenommen werden. Es wird aber bevorzugt, daß die Messung an einer Stelle vorgenommen wird, an der die maximale Konzentration der zu entfernenden Substanz in der Dialyselösung vorliegt. Dies dient dazu, die zuverlässigsten Messwerte der jeweiligen Konzentration erhalten zu können. Dies ist für gewöhnlich im Ausflussteil auf der Dialyseseite der Fall. Insbesondere bevorzugt ist die Messung am Eingang des Ausflussteiles, da beim Durchlaufen der Flüssigkeit durch das Ausflussteil die Konzentration nicht mehr erhöht wird, aber im weiteren Verlauf bereits Diffusionseffekte innerhalb des Ausflussteiles auftreten können, die die Messung beeinflussen können.

Bei der Anbindung des Ausflussteiles an den Dialysefilter hat es sich als vorteilhaft erwiesen, daß keine harten Kanten oder abrupten Verengungen des Flussweges auftreten. Es soll ein möglichst laminares Strömungsprofil, wie es für gewöhnlich in dem Dialysefilter vorliegt, erhalten bleiben. Andernfalls könnten an Kanten und Engstellungen Turbulenzen auftreten, was an diesen Stellen zu einer Ungleichverteilung der transportierten Stoffe und damit potentiell zu nicht exakten Messwerten führen kann. Da diese Turbulenzen anhängig von der regulierbaren Strömungsgeschwindigkeit sind und davon auszugehen ist, daß diese Konzentrationsänderungen nicht-linear sein können, können Turbulenzen eine Fehlerquelle darstellen. Handelsübliche Dialyseschläuche erfüllen dieses Kriterium.

Als regelbare Größen an einer Blutbehandlungseinheit sind bei Hämodialysegeräten insbesondere der Fluss des Blutes und der Fluss der Dialyselösung von Bedeutung. Desweiteren kann beispielsweise über die Bilanzkammer die Ultrafiltrationsrate bzw. der angelegte transmembrane Druck, die Behandlungsdauer des Patienten angepasst werden und die Geschwindigkeit (das Umschaltintervall der Bilanzkammer) und das Maß der Volumenkompensation geregelt werden.

Da die Hämodiafiltration eine Kombination von Hämodialyse und Hämofiltration darstellt, sind die gleichen Parameter regelbar wie bei den Einzelverfahren einstellbar.

Das erfindungsgemäße Verfahren kann grundsätzlich an allen Säugetieren durchgeführt werden. Bevorzugt wird jedoch eine Verwendung bei der Behandlung menschlicher Patienten.

Kritische physiologische Größen, die einer unbegrenzten Regelbarkeit des Dialyseprozesses entgegenstehen, sind Blutfluss und Ultrafiltrationsrate.

Daher werden in bevorzugten Ausführungsformen der Hämatokritwert und/oder die Plasmaproteinkonzentration durch geeignete Zusatzverfahren ermittelt und können in die Berechnung der optimalen Dialysegeräteinstellungen als begrenzende Größen (beispielsweise für die Ultrafiltrationsrate) einfließen. Es kann erfindungsgemäß ein Abgleich mit zuvor gespeicherten Literaturwerten stattfinden oder es können individuell für jeden Dialysegerättyp, Einzelgerät und Patient Werte über eine oder mehrere Dialysen als Vergleichswerte gespeichert und zur Bewertung herangezogen werden.

Eine häufiger auftretende Problematik sind hypotensive Episoden bei Patienten mit einer dementsprechenden Vulnerabilität. Dies kann bis zu einem Kreislaufversagen führen. Daher wird bei manchen Dialysegeräten der Blutdruck automatisch kontrolliert. Bei manchen Geräten wird bei Unterschreiten eines kritischen Wertes ein Alarm ausgelöst. In der Regel wird daraufhin mit Aussetzen der Ultrafiltration reagiert oder mit einer Herabsetzung des Blutflusses. In besonders kritischen Fällen wird der Fluss der Dialyselösung angehalten. Aufgrund der fehlenden Diffusion wird die Reinigungsleistung dadurch extrem gemindert.

Wenn durch die Patientenhistorie bekannt ist, daß bei einer entsprechend hohen Kombination von Blutfluss und Dialyselösungsfluss hypotensive Episoden aufgetreten sind, bilden die somit bekannten kritischen Werte eine Obergrenze, unterhalb derer die Regulierung der beiden Flüsse stattfindet. Sollte die Auswertung der photospektroskopischen Messung in diesem Fall Flüsse oberhalb der bekannten kritischen Werte empfehlen, wird durch die Einbeziehung der Patientenhistorie dafür gesorgt, daß die Flüsse unterhalb der kritischen Werte bleiben.

Natürliche Grenzen der Regelbarkeit der beiden Flüsse bei der Hämodialyse sind daher einerseits durch die technischen Möglichkeiten des jeweiligen Dialysators sowie Stoffeigenschaften beispielsweise der Dialyseschläuche gegeben. Andererseits können sich auch zu hohe Geschwindigkeiten des Blutflusses negativ auswirken. Auch auf der Blutseite können Turbulenzen entstehen und Scherkräfte wirken. Die physiologisch möglichen extrakorporalen Blutflüsse sind aber meistens gering genug, so daß keine großen Scherkräfte auftreten.

Die beiden Flüsse für das Blut $Q_B$ und die Dialyselösung $Q_D$ können in einen Quotient $Q_D/Q_B$ zueinander gesetzt werden.

Erfindungsgemäß können je nach dem Quotienten $Q_D/Q_B$ drei verschiedene Modi der Dialyseeffizienz definiert werden:

    a) Eco Mode: $0,5 < Q_O/Q_B < 1,3$
    b) Efficiency Mode: $1,3 \leq Q_D/Q_B \leq 2,0$
    c) Power Mode: $4,0 > Q_D/Q_B > 2,0$

oder durch den Kehrwert ausgedrückt:

a) Eco Mode: $2,0 > Q_B/Q_D > 0,77$
b) Efficiency Mode: $0,5 \leq Q_B/Q_D \leq 0,77$
c) Power Mode: $0,25 < Q_B/Q_D < 0,5$

Gleiches gilt auch für die über die Therapie verwendeten Volumina, die zu Beginn einer Dialysesitzung festgelegt wurden. Das Verhältnis zwischen Blutvolumen $V_B$ und Volumen an Dialysatflüssigkeit $V_D$ kann daher in weiteren Ausführungsformen verwendet werden.

a) Eco Mode: $0,5 < V_D/V_B < 1,3$
b) Efficiency Mode: $1, 3 \leq VD/VB \leq 2,0$
c) Power Mode: $4,0 > V_D/V_B > 2,0$

oder durch den Kehrwert ausgedrückt:

a) Eco Mode: $2,0 > V_B/V_D > 0,77$
b) Efficiency Mode: $0,5 \leq V_B/V_D \leq 0,77$
c) Power Mode: $0,25 < V_B/V_D < 0,5$

Da die zu den jeweiligen Volumina-basierten Modi angegebenen Bereiche identisch zu denen der Flüsse $Q_B$ und $Q_D$ sind, wird in der weiteren Beschreibung der Anwendungsmodi auf eine Unterscheidung verzichtet. Erfindungsgemäß bezieht sich der Begriff Anwendungsmodi somit auf beide Einteilungskriterien für die Modi.

Der Eco Mode (Economy Mode) zeichnet sich dadurch aus, daß $Q_B$ ($V_B$) nicht wesentlich größer als $Q_D$ ($V_D$) ist. Dies bedeutet, daß vergleichsweise wenig Dialyselösung verbraucht wird, um ein qualitativ immer noch gutes Dialyseergebnis zu erzielen. Eine beträchtliche Menge an Dialyselösung sowie deren Entsorgung kann eingespart werden. Wenn der Schwerpunkt der Dialyse auf einem möglichst wirtschaftlichem Betrieb liegt, ist der Eco Mode besonders bevorzugt.

Der Efficiency Mode bildet einen Mittelbereich des Quotienten $Q_D/Q_B$ ($V_D/V_B$). In diesem Bereich wird der bestmögliche Kompromiss aus Wirtschaftlichkeit und optimalem Dialyseergebnis gesucht. Für die Mehrzahl der Anwendungen wird dieser Modus besonders bevorzugt sein.

Der Power Mode ist durch einen besonders hohen Fluss der Dialyselösung im Vergleich zum Blutfluss gekennzeichnet. In diesem Modus wird eine optimale oder nahezu optimale Reinigungsleistung erzielt. Allerdings wird dafür überproportional viel Dialyselösung verbraucht. Dieser Modus wird besonders bevorzugt, wenn aufgrund auffällig hoher Messwerte für Harnsäure (und indirekt damit für Harnstoff), durch ein akutes Nierenversagen (beispielsweise bei Multiorganversagen oder nach einer Operation) oder bei akuten Vergiftungen es medizinisch indiziert ist, so schnell wie möglich eine weitestgehende Reinigung des Blutes zu erreichen. Die Kostenfrage tritt hierbei in den Hintergrund.

Es sei angemerkt, daß erfindungsgemäß auch weitere Operationsmodi anhand des Quotienten $Q_D/Q_B$ ($V_D/V_B$) sowie des jeweiligen Kehrwertes bestimmt werden können und miteinander kombiniert werden können. Die Grenzziehung bezüglich der Werte von $Q_D/Q_B$ ($V_D/V_B$) sowie des jeweiligen Kehrwertes und die Benennung der einzelnen Operationsmodi kann daher erfindungsgemäß variieren.

Erfindungsgemäß kann vom Anwender an einer zentralen Recheneinheit vorgegeben werden, in welchem Modus eine Steuerung vorzugsweise stattfinden soll. Die zentrale Recheneinheit prüft, ob die aktuell eingehenden Messwerte und die sich daraus ergebenden Anpassungen des Dialysegerätes mit den für diesen Steuerungsmodus hinterlegten Werten in Übereinstimmung zu bringen ist. Im positiven Fall erfolgt eine Anpassung des Dialysegerätes, insbesondere der Flüsse des Bluts und der Dialyselösung, innerhalb des für diesen Modus vorgesehenen Bereiches für den Quotienten $Q_D/Q_B$ bzw. des Kehrwertes. Im negativen Fall zeigt die zentrale Recheneinheit einen Vorschlag an, in welchen Modus das Dialysegerät umgeschaltet werden soll. Wahlweise kann auch eine automatische Umstellung erfolgen.

Die zentrale Recheneinheit kann jedes Gerät sein, daß über eine CPU, eine Anzeigevorrichtung und eine Eingabevorrichtung verfügt, beispielsweise ein Computer. Die zentrale Recheneinheit kann an dem Dialysegerät selbst angebracht sein oder unabhängig davon betrieben werden. Gleiches gilt für eine Anzeigevorrichtung für die ermittelten Messwerte. Deren Anzeige kann über die Anzeigevorrichtung an der zentralen Recheneinheit oder über eine eigene Anzeigevorrichtung erfolgen. Die Übertragung der photospektroskopischen Messwerte kann über alle im Stand der Technik bekannten Vorrichtungen erfolgen, beispielsweise über eine Kabelverbindung oder eine WLAN-Verbindung.

Der Verlauf der Reinigungsleistung kann mit einem auf der zentralen Recheneinheit hinterlegten modellhaften Verlauf verglichen werden und eine entsprechende Korrekturmaßnahme kann vorgeschlagen oder vorgenommen werden.

Für jeden Patienten können die bei seinen Dialysesitzungen ermittelten Messwerte und Flüsse auf einem Datenträger festgehalten werden und zur Wiederverwendung zur Verfügung gestellt werden.

# EP 2 621 550 B1

In anderen Ausführungsformen können auch die baubedingten Dialysatorspezifikationen zur Optimierung des Blutreinigungsprozesses herangezogen werden. Hierbei können auch die patientenspezifischen Daten und die Dialysatorspezifikationen kombiniert werden.

Die erhobenen Daten können zur Erstellung von Flussprofilen verwendet werden, die wiederum dem Anwender vorgeschlagen werden.

Auf der Anzeigevorrichtung können der laufende Optimierungsprozess dem Anwender angezeigt werden. Diese Daten können auf einem Datenträger gespeichert werden. In gleicher Weise können die eingesparte Menge an Dialyselösung und/oder die gesteigerte Dialysequalität angezeigt werden.

Dementsprechend umfasst das erfindungsgemäße Verfahren zur Optimierung des Verbrauchs an Dialyseflüssigkeit in einer Blutbehandlungseinheit die folgenden Schritte:

a) Vorauswahl eines Operationsmodus und eines untersten akzeptablen Werts für einen Dialyseleistungsparameter;

b) Ermittlung der aktuellen Messwerte für die Flüsse des Blutes und der Dialyseflüssigkeit in der Blutbehandlungseinheit;

c) Messung der UV-Absorbanz mindestens einer harnpflichtigen Substanz im Dialysatausfluss oder im extrakorporalen Blutkreislauf der Blutbehandlungseinheit;

d) Weiterleitung der Messwerte der UV-Absorbanz und der aktuellen Messwerte für den Fluss des Blutes und der Dialyseflüssigkeit an eine zentrale Recheneinheit;

e) Ermittlung eines auf den Verbrauch an Dialyseflüssigkeit optimierten Flusses der Dialyselösung, und/oder des Blutes und/oder eines relativen Verhältnisses der beiden Flüsse innerhalb eines für den ausgewählten Operationsmodus festgelegten $Q_D/Q_B$-Bereichs in der zentralen Recheneinheit, ohne daß infolgedessen der unterste akzeptable Wert des ausgewählten Dialyseleistungsparameters unterschritten wird;

oder

e') Ermittlung eines auf den Verbrauch an Dialyseflüssigkeit optimierten Flusses der Dialyselösung, und/oder des Blutes und/oder eines relativen Verhältnisses des Voluminadurchsatzes der Dialyseflüssigkeit und des Blutes innerhalb eines für den ausgewählten Operationsmodus festgelegten $V_D/V_B$-Bereichs in der zentralen Recheneinheit, ohne daß infolgedessen der unterste akzeptable Wert des ausgewählten Dialyseleistungsparameters unterschritten wird;

f) Anzeigen der ermittelten Änderung des Flusses der Dialyselösung und/oder des Blutes an der zentralen Recheneinheit und/oder automatische Nachregelung gemäß der berechneten Änderung an der Blutbehandlungseinheit; und

g) Wiederholung der Schritte b) bis f) nach jeweils einem zuvor festgelegten Zeitintervall oder Zeitintervallschema bis zur Beendigung der Dialysesitzung.

Die Schritte werden in der angegebenen Reihenfolge von a) bis g) durchlaufen. Im vorgenannten Verfahren sind die Schritte e) und e') Alternativen, d.h. das erfindungsgemäße Verfahren umfasst die Schritte a) bis g) oder a) bis d), e'), f) und g).

Die vorliegende Erfindung betrifft somit unter anderem die folgenden Verfahren. Verfahren zur Optimierung des Verbrauchs an Dialyseflüssigkeit in einer Blutbehandlungseinheit die folgenden Schritte:

a) Vorauswahl eines Operationsmodus und eines untersten akzeptablen Werts für einen Dialyseleistungsparameter;

b) Ermittlung der aktuellen Messwerte für die Flüsse des Blutes und der Dialyseflüssigkeit in der Blutbehandlungseinheit;

c) Messung der UV-Absorbanz mindestens einer harnpflichtigen Substanz im Dialysatausfluss oder im extrakorporalen Blutkreislauf der Blutbehandlungseinheit;

d) Weiterleitung der Messwerte der UV-Absorbanz und der aktuellen Messwerte für den Fluss des Blutes und der Dialyseflüssigkeit an eine zentrale Recheneinheit;

e) Ermittlung eines auf den Verbrauch an Dialyseflüssigkeit optimierten Flusses der Dialyselösung, und/oder des Blutes und/oder eines relativen Verhältnisses der beiden Flüsse innerhalb eines für den ausgewählten Operationsmodus festgelegten $Q_D/Q_B$-Bereichs in der zentralen Recheneinheit, ohne daß infolgedessen der unterste akzeptable Werts des ausgewählten Dialyseleistungsparameters unterschritten wird;

f) Anzeigen der ermittelten Änderung des Flusses der Dialyselösung und/oder des Blutes an der zentralen Recheneinheit und/oder automatische Nachregelung gemäß der berechneten Änderung an der Blutbehandlungseinheit; und

g) Wiederholung der Schritte b) bis f) nach jeweils einem zuvor festgelegten Zeitintervall oder Zeitintervallschema bis zur Beendigung der Dialysesitzung.

Wenn das Volumen der Dialyselösung variiert werden soll, stellt sich das erfindungsgemäße Verfahren wie folgt dar:

a) Vorauswahl eines Operationsmodus und eines untersten akzeptablen Werts für einen Dialyseleistungsparameter;

b) Ermittlung der aktuellen Messwerte für den Fluss des Blutes und der Dialyseflüssigkeit in der Blutbehandlungseinheit;

c) Messung der UV-Absorbanz mindestens einer harnpflichtigen Substanz im Dialysatausfluss oder im extrakorporalen Blutkreislauf der Blutbehandlungseinheit;

d) Weiterleitung der Messwerte der UV-Absorbanz und der aktuellen Messwerte für den Fluss des Blutes und der Dialyseflüssigkeit an eine zentrale Recheneinheit;

e') Ermittlung eines auf den Verbrauch an Dialyseflüssigkeit optimierten Flusses der Dialyselösung, und/oder des Blutes und/oder eines relativen Verhältnisses des Voluminadurchsatzes der Dialyseflüssigkeit und des Blutes innerhalb eines für den ausgewählten Operationsmodus festgelegten $V_D/V_B$-Bereichs in der zentralen Recheneinheit, ohne daß infolgedessen der unterste akzeptable Werts des ausgewählten Dialyseleistungsparameters unterschritten wird;

f) Anzeigen der ermittelten Änderung des Flusses der Dialyselösung und/oder des Blutes an der zentralen Recheneinheit und/oder deren automatische Nachregelung gemäß der berechneten Änderung an der Blutbehandlungseinheit; und

g) Wiederholung der Schritte b) bis f) nach jeweils einem zuvor festgelegten Zeitintervall oder Zeitintervallschema bis zur Beendigung der Dialysesitzung.

Wird ein bestimmtes zur Verfügung stehendes Volumen an Dialyseflüssigkeit vorgegeben, so ergibt sich folgendes erfindungsgemäßes Verfahren:

a) Vorauswahl eines Operationsmodus und eines untersten akzeptablen Werts für einen Dialyseleistungsparameter;

a') Vorauswahl eines bei der Dialysesitzung zu verbrauchenden Volumens an Dialyseflüssigkeit;

b) Ermittlung der aktuellen Messwerte für den Fluss des Blutes und der Dialyseflüssigkeit in der Blutbehandlungseinheit;

c) Messung der UV-Absorbanz mindestens einer harnpflichtigen Substanz im Dialysatausfluss oder im extrakorporalen Blutkreislauf der Blutbehandlungseinheit;

d) Weiterleitung der Messwerte der UV-Absorbanz und der aktuellen Messwerte für den Fluss des Blutes und der Dialyseflüssigkeit an eine zentrale Recheneinheit;

e") Ermittlung eines auf den ausgewählten Dialyseleistungsparameter optimierten Flusses oder optimierten Abfolgeschemas der Flüsse der Dialyselösung, und/oder des Blutes und/oder eines relativen Verhältnisses der beiden Flüsse innerhalb eines für den ausgewählten Operationsmodus festgelegten $Q_D/Q_B$- oder $V_D/V_B$-Bereichs in der zentralen Recheneinheit unter Wahrung des bei der Dialysesitzung zu verbrauchenden Volumens an Dialyseflüssigkeit;

f) Anzeigen der ermittelten Änderung der Flüsse der Dialyselösung und/oder des Blutes an der zentralen Recheneinheit und/oder automatische Nachregelung gemäß der berechneten Änderung an der Blutbehandlungseinheit; und

g) Wiederholung der Schritte b) bis f) nach jeweils einem zuvor festgelegten Zeitintervall oder Zeitintervallschema bis zur Beendigung der Dialysesitzung.

Der Schritt a') ist im Vergleich zu den vorgenannten Verfahren hinzugekommen und Schritt e") tritt anstelle von Schritt e) oder von Schritt e'), wobei Schritt e") eine Alternative hinsichtlich des $Q_D/Q_B$-Bereichs und des $V_D/V_B$-Bereichs enthält. Die beiden Verfahren, welche durch diese Alternative abgedeckt werden umfassen die folgenden Schritte: a), a'), b) - d), gefolgt von Schritt e):

e) Ermittlung eines auf den ausgewählten Dialyseleistungsparameter optimierten Flusses oder optimierten Abfolgeschemas der Flüsse der Dialyselösung, und/oder des Blutes und/oder eines relativen Verhältnisses der beiden Flüsse innerhalb eines für den ausgewählten Operationsmodus festgelegten $Q_D/Q_B$-Bereichs in der zentralen Recheneinheit unter Wahrung des bei der Dialysesitzung zu verbrauchenden Volumens an Dialyseflüssigkeit;

und Schritte f) und g).
Das alternative Verfahren umfasst die folgenden Schritte:

a), a'), b) - d), gefolgt von Schritt e'):

e') Ermittlung eines auf den ausgewählten Dialyseleistungsparameter optimierten Flusses oder optimierten Abfolgeschemas der Flüsse der Dialyselösung, und/oder des Blutes und/oder eines relativen Verhältnisses der beiden Flüsse innerhalb eines für den ausgewählten Operationsmodus festgelegten $V_D/V_B$-Bereichs in der zentralen Recheneinheit unter Wahrung des bei der Dialysesitzung zu verbrauchenden Volumens an Dialyseflüssigkeit;

und Schritte f) und g).

Wenn der Fluss der Dialyselösung variiert werden soll, stellt sich das erfindungsgemäße Verfahren wie folgt dar:

a) Vorauswahl eines Operationsmodus und eines untersten akzeptablen Werts für einen Dialyseleistungsparameter;
b) Ermittlung der aktuellen Messwerte für die Flüsse des Blutes und der Dialyseflüssigkeit in der Blutbehandlungseinheit;
c) Variation der Flüsse der Dialyselösung in der Blutbehandlungseinheit während einer Dialysesitzung;
d) Messung der UV-Absorbanz mindestens einer harnpflichtigen Substanz im Dialysatausfluss oder im extrakorporalen Blutkreislauf der Blutbehandlungseinheit;
e) Weiterleitung der Messwerte der UV-Absorbanz und der aktuellen Messwerte für den Fluss des Blutes und der Dialyseflüssigkeit an eine zentrale Recheneinheit;
f) Ermittlung eines auf den Verbrauch an Dialyseflüssigkeit optimierten Flusses der Dialyselösung, und/oder des Blutes und/oder eines relativen Verhältnisses der beiden Flüsse innerhalb eines für den ausgewählten Operationsmodus festgelegten $Q_D/Q_B$-Bereichs in der zentralen Recheneinheit, ohne daß infolgedessen der unterste akzeptable Werts des ausgewählten Dialyseleistungsparameters unterschritten wird;
g) Anzeigen der ermittelten Änderung der Flüsse der Dialyselösung und/oder des Blutes an der zentralen Recheneinheit und/oder automatische Nachregelung gemäß der berechneten Änderung an der Blutbehandlungseinheit; und
h) Wiederholung der Schritte b) bis g) nach jeweils einem zuvor festgelegten Zeitintervall oder Zeitintervallschema bis zur Beendigung der Dialysesitzung.

Wenn bei der vorgenannten Ausführungsform der Fluss der Dialyselösung variiert werden soll, stellt sich das erfindungsgemäße Verfahren wie folgt dar:

a) Vorauswahl eines Operationsmodus und eines untersten akzeptablen Werts für einen Dialyseleistungsparameter;
b) Ermittlung der aktuellen Messwerte für den Fluss des Blutes und der Dialyseflüssigkeit in der Blutbehandlungseinheit;
c) Variation des Flusses der Dialyselösung in der Blutbehandlungseinheit während einer Dialysesitzung;
d) Messung der UV-Absorbanz mindestens einer harnpflichtigen Substanz im Dialysatausfluss oder im extrakorporalen Blutkreislauf der Blutbehandlungseinheit;
e) Weiterleitung der Messwerte der UV-Absorbanz und der aktuellen Messwerte für den Fluss des Blutes und der Dialyseflüssigkeit an eine zentrale Recheneinheit;
f) Ermittlung eines auf den Verbrauch an Dialyseflüssigkeit optimierten Flusses der Dialyselösung, und/oder des Blutes und/oder eines relativen Verhältnisses des Voluminadurchsatzes der Dialyseflüssigkeit und des Blutes innerhalb eines für den ausgewählten Operationsmodus festgelegten $V_D/V_B$-Bereichs in der zentralen Recheneinheit, ohne daß infolgedessen der unterste akzeptable Werts des ausgewählten Dialyseleistungsparameters unterschritten wird;
g) Anzeigen der ermittelten Änderung des Flusses der Dialyselösung und/oder des Blutes an der zentralen Recheneinheit und/oder deren automatische Nachregelung gemäß der berechneten Änderung an der Blutbehandlungseinheit; und
h) Wiederholung der Schritte b) bis g) nach jeweils einem zuvor festgelegten Zeitintervall oder Zeitintervallschema bis zur Beendigung der Dialysesitzung.

Das Verfahren zur Optimierung des Einsatzes eines Volumens an Dialyseflüssigkeit in einer Blutbehandlungseinheit umfasst erfindungsgemäß die folgenden Schritte :

a) Vorauswahl eines Operationsmodus und eines untersten akzeptablen Werts für einen Dialyseleistungsparameter;
a') Vorauswahl eines bei der Dialysesitzung zu verbrauchenden Volumens an Dialyseflüssigkeit;
b) Ermittlung der aktuellen Messwerte für den Fluss des Blutes und der Dialyseflüssigkeit in der Blutbehandlungseinheit;
c) Messung der UV-Absorbanz mindestens einer harnpflichtigen Substanz im Dialysatausfluss oder im extrakorporalen Blutkreislauf der Blutbehandlungseinheit;
d) Weiterleitung der Messwerte der UV-Absorbanz und der aktuellen Messwerte für den Fluss des Blutes und der Dialyseflüssigkeit an eine zentrale Recheneinheit;
e") Ermittlung eines auf den ausgewählten Dialyseleistungsparameter optimierten Abfolgeschemas der Flüsse der Dialyselösung, und/oder des Blutes und/oder eines relativen Verhältnisses der beiden Flüsse innerhalb eines für den ausgewählten Operationsmodus festgelegten $Q_D/Q_B$- oder $V_D/V_B$-Bereichs in der zentralen Recheneinheit unter Wahrung des bei der Dialysesitzung zu verbrauchenden Volumens an Dialyseflüssigkeit;

f) Anzeigen der ermittelten Änderung der Flüsse der Dialyselösung und/oder des Blutes an der zentralen Recheneinheit und/oder automatische Nachregelung gemäß der berechneten Änderung an der Blutbehandlungseinheit; und

g) Wiederholung der Schritte b) bis f) nach jeweils einem zuvor festgelegten Zeitintervall oder Zeitintervallschema bis zur Beendigung der Dialysesitzung.

Wenn bei der vorgenannten Ausführungsform der Fluss der Dialyselösung variiert werden soll, stellt sich das erfindungsgemäße Verfahren zur Optimierung des Einsatzes eines Volumens an Dialyseflüssigkeit in einer Blutbehandlungseinheit wie folgt dar:

a) Vorauswahl eines Operationsmodus und eines untersten akzeptablen Werts für einen Dialyseleistungsparameter;

a') Vorauswahl eines bei der Dialysesitzung zu verbrauchenden Volumens an Dialyseflüssigkeit;

b) Ermittlung der aktuellen Messwerte für den Fluss des Blutes und der Dialyseflüssigkeit in der Blutbehandlungseinheit;

c) Variation des Flusses der Dialyselösung in der Blutbehandlungseinheit während einer Dialysesitzung;

d) Messung der UV-Absorbanz mindestens einer harnpflichtigen Substanz im Dialysatausfluss oder im extrakorporalen Blutkreislauf der Blutbehandlungseinheit;

e) Weiterleitung der Messwerte der UV-Absorbanz und der aktuellen Messwerte für den Fluss des Blutes und der Dialyseflüssigkeit an eine zentrale Recheneinheit;

f) Ermittlung eines auf den ausgewählten Dialyseleistungsparameter optimierten Abfolgeschemas der Flüsse der Dialyselösung, und/oder des Blutes und/oder eines relativen Verhältnisses der beiden Flüsse innerhalb eines für den ausgewählten Operationsmodus festgelegten $Q_D/Q_B$- oder $V_D/V_B$-Bereichs in der zentralen Recheneinheit unter Wahrung des bei der Dialysesitzung zu verbrauchenden Volumens an Dialyseflüssigkeit;

g) Anzeigen der ermittelten Änderung der Flüsse der Dialyselösung und/oder des Blutes an der zentralen Recheneinheit und/oder automatische Nachregelung gemäß der berechneten Änderung an der Blutbehandlungseinheit; und

h) Wiederholung der Schritte b) bis g) nach jeweils einem zuvor festgelegten Zeitintervall oder Zeitintervallschema bis zur Beendigung der Dialysesitzung.

Als bevorzugter Dialyseleistungsparameter kann der Kt/V verwendet werden. Als bevorzugte harnpflichtige Substanz kann Harnsäure verwendet werden.

Das erfindungsgemäße Verfahren bei Verwendung von Harnsäure und Kt/V stellt sich wie folgt dar:

a) Vorauswahl eines Operationsmodus und eines untersten akzeptablen Werts für den Kt/V;

b) Ermittlung der aktuellen Messwerte für die Flüsse des Blutes und der Dialyseflüssigkeit in der Blutbehandlungseinheit;

c) Messung der UV-Absorbanz von Harnsäure im Dialysatausfluss oder im extrakorporalen Blutkreislauf der Blutbehandlungseinheit;

d) Weiterleitung der Messwerte der UV-Absorbanz und der aktuellen Messwerte für den Fluss des Blutes und der Dialyseflüssigkeit an eine zentrale Recheneinheit;

e) Ermittlung eines auf den Verbrauch an Dialyseflüssigkeit optimierten Flusses der Dialyselösung, und/oder des Blutes und/oder eines relativen Verhältnisses der beiden Flüsse innerhalb eines für den ausgewählten Operationsmodus festgelegten $Q_D/Q_B$-Bereichs in der zentralen Recheneinheit, ohne daß infolgedessen der unterste akzeptable Wert des Kt/V unterschritten wird;

oder

e') Ermittlung eines auf den Verbrauch an Dialyseflüssigkeit optimierten Flusses der Dialyselösung, und/oder des Blutes und/oder eines relativen Verhältnisses des Voluminadurchsatzes der Dialyseflüssigkeit und des Blutes innerhalb eines für den ausgewählten Operationsmodus festgelegten $V_D/V_B$-Bereichs in der zentralen Recheneinheit, ohne daß infolgedessen der unterste akzeptable Wert des Kt/V unterschritten wird;

f) Anzeigen der ermittelten Änderung des Flusses der Dialyselösung und/oder des Blutes an der zentralen Recheneinheit und/oder automatische Nachregelung gemäß der berechneten Änderung an der Blutbehandlungseinheit; und

g) Wiederholung der Schritte b) bis f) nach jeweils einem zuvor festgelegten Zeitintervall oder Zeitintervallschema bis zur Beendigung der Dialysesitzung.

Sowohl die Zusammensetzung und die Fließeigenschaften des Blutes des Patienten, der ausgewählte Typ des Dialysegeräts und sein Zustand sowie vor allem der aktuelle Zustand der Dialysatormembran tragen zu der Vielfalt und Unvorhersehbarkeit der zu ermittelnden Zustände und der sich daraus ergebenden Optimierungsmöglichkeiten und Änderungsvorschläge bei. Es ist daher intuitiv ersichtlich, daß die Optimierungsmöglichkeiten und Änderungsvorschläge sich nicht aus einer Generalformel ergeben können, sondern für jeden Fall individuell ermittelt werden müssen. Hierfür wird auf die Empirie zurückgegriffen. Entsprechende Werte können aus dem bisherigen Verlauf der Dialysesitzung, früheren Dialysesitzungen des jeweiligen Patienten an diesem Dialysegerät, der Patientenhistorie und den Dialysatorspezifikationen ermittelt werden. Eine wichtige Rolle spielt dabei die zuvor beschriebene Variation des Flusses der Dialyselösung während einer Dialysesitzung. Je mehr empirische Werte vorliegen, desto veriässlicher läßt sich der Verbrauch an Dialyseflüssigkeit optimieren durch eine geeignete Anpassung des Flusses der Dialyselösung, und/oder des Blutes und/oder eines relativen Verhältnisses der beiden Flüsse. Analog läßt sich derart ein optimierten Abfolgeschemas der Flüsse der Dialyselösung, und/oder des Blutes und/oder eines relativen Verhältnisses der beiden Flüsse zueinander ermitteln.

Gleichermaßen bezieht sich die vorliegende Erfindung auf Vorrichtungen, die geeignet sind, die zuvor beschriebenen erfindungsgemäßen Verfahren zu implementieren. Insbesondere bezieht sich die vorliegende Erfindung daher auf eine

Vorrichtung zur Blutbehandlung, die

eine Blutbehandlungseinheit,
eine UV-Messvorrichtung und
eine zentrale Recheneinheit umfasst,

wobei die zentrale Recheneinheit über Mittel verfügt, die aus den von der UV-Messvorrichtung gemessenen Werten der UV-Absorption im Dialysatausfluss ermittelten Wert gemäß den erfindungsgemäßen Verfahren zur Optimierung des Verbrauchs an Dialyseflüssigkeit in einer Blutbehandlungseinheit einen ausgewählten Dialyseleistungsparameter berechnet und aufgrund dessen eine auf den Verbrauch an Dialyselösung optimierte Einstellung der Flüsse des Blutes und der Dialyseflüssigkeit ermittelt,
und über Mittel verfügt, diese optimierte Einstellung anzuzeigen und/oder automatisch an der Blutbehandlungseinheit vorzunehmen.

Anders gesagt umfasst die erfindungsgemäße Vorrichtung zur Blutbehandlung eine Blutbehandlungseinheit, eine UV-Messvorrichtung und eine zentrale Recheneinheit, wobei die zentrale Recheneinheit über Mittel verfügt, die ein Verfahren gemäß einem der Ansprüche 1-19 ausführen kann wie beispielsweise eine Vorrichtung zur Blutbehandlung, die

eine Blutbehandlungseinheit,
eine UV-Messvorrichtung und
eine zentrale Recheneinheit umfasst,

wobei die zentrale Recheneinheit über Mittel verfügt, die ein Verfahren implementieren können, das die folgenden Schritte umfasst:

a) Vorauswahl eines Operationsmodus und eines untersten akzeptablen Werts für einen Dialyseleistungsparameter;
b) Ermittlung der aktuellen Messwerte für die Flüsse des Blutes und der Dialyseflüssigkeit in der Blutbehandlungseinheit;
c) Messung der UV-Absorbanz mindestens einer harnpflichtigen Substanz im Dialysatausfluss oder im extrakorporalen Blutkreislauf der Blutbehandlungseinheit;
d) Weiterleitung der Messwerte der UV-Absorbanz und der aktuellen Messwerte für den Fluss des Blutes und der Dialyseflüssigkeit an eine zentrale Recheneinheit;
e) Ermittlung eines auf den Verbrauch an Dialyseflüssigkeit optimierten Flusses der Dialyselösung, und/oder des Blutes und/oder eines relativen Verhältnisses der beiden Flüsse innerhalb eines für den ausgewählten Operationsmodus festgelegten $Q_D/Q_B$-Bereichs in der zentralen Recheneinheit, ohne daß infolgedessen der unterste akzeptable Wert des ausgewählten Dialyseleistungsparameters unterschritten wird;
oder
e') Ermittlung eines auf den Verbrauch an Dialyseflüssigkeit optimierten Flusses der Dialyselösung, und/oder des Blutes und/oder eines relativen Verhältnisses des Voluminadurchsatzes der Dialyseflüssigkeit und des Blutes innerhalb eines für den ausgewählten Operationsmodus festgelegten $V_D/V_B$-Bereichs in der zentralen Recheneinheit, ohne daß infolgedessen der unterste akzeptable Wert des ausgewählten Dialyseleistungsparameters unterschritten wird;
f) Anzeigen der ermittelten Änderung des Flusses der Dialyselösung und/oder des Blutes an der zentralen Recheneinheit und/oder automatische Nachregelung gemäß der berechneten Änderung an der Blutbehandlungseinheit; und
g) Wiederholung der Schritte b) bis f) nach jeweils einem zuvor festgelegten Zeitintervall oder Zeitintervallschema bis zur Beendigung der Dialysesitzung.

EP 2 621 550 B1

Gleichermaßen bezieht sich die vorliegende Erfindung auf eine Vorrichtung zur Blutbehandlung, die

eine Blutbehandlungseinheit,
eine UV-Messvorrichtung und
eine zentrale Recheneinheit umfasst,

wobei die zentrale Recheneinheit über Mittel verfügt, die aus den von der UV-Messvorrichtung gemessenen Werten der UV-Absorption im Dialysatausfluss ermittelten Wert gemäß dem erfindungsgemäßen Verfahren zur Optimierung des Einsatzes eines vorgegebenen Volumens an Dialyseflüssigkeit in einer Blutbehandlungseinheit einen ausgewählten Dialyseleistungsparameter berechnet und aufgrund dessen eine auf den Einsatz eines vorgegebenen Volumens an Dialyseflüssigkeit optimierte Einstellung der Flüsse des Blutes und der Dialyseflüssigkeit ermittelt, und über Mittel verfügt, diese optimierte Einstellung anzuzeigen und/oder automatisch an der Blutbehandlungseinheit vorzunehmen.

Anders gesagt umfasst die erfindungsgemäße Vorrichtung zur Blutbehandlung eine Blutbehandlungseinheit, eine UV-Messvorrichtung und eine zentrale Recheneinheit, wobei die zentrale Recheneinheit über Mittel verfügt, die ein Verfahren gemäß dem Anspruch 2 implementieren können.

Bei den besagten Mitteln, die aus den von der UV-Messvorrichtung gemessenen Werten der UV-Absorption im Dialysatausfluss ermittelten Wert gemäß dem erfindungsgemäßen Verfahren zur Optimierung des Einsatzes eines vorgegebenen Volumens an Dialyseflüssigkeit in einer Blutbehandlungseinheit und/oder dem erfindungsgemäßen Verfahren zur Optimierung des Einsatzes eines vorgegebenen Volumens an Dialyseflüssigkeit in einer Blutbehandlungseinheit einen ausgewählten Dialyseleistungsparameter berechnen und aufgrund dessen eine auf den Einsatz eines vorgegebenen Volumens an Dialyseflüssigkeit optimierte Einstellung der Flüsse des Blutes und der Dialyseflüssigkeit ermitteln, sowie den Mitteln, die diese optimierte Einstellung anzeigen und/oder automatisch an der Blutbehandlungseinheit vornehmen, handelt es sich um aus dem Stand der Technik bekannte Hardware-Komponenten der zentralen Recheneinheit (in der Regel einem PC) sowie dafür speziell angefertigter Software.

Die Begriffe "baubedingte Dialysatorspezifikationen" oder "Dialysatorspezifikationen" beziehen sich bei der vorliegenden Erfindung auf die technischen Einzelheiten und Zusammenhänge, die sich aus der produktionsbedingten Bauweise des jeweiligen Dialysegerätes ergeben und durch den funktionsgemäßen Gebrauch des Dialysegerätes nicht verändert werden können. Sie setzen die Rahmenbedingungen, innerhalb derer Einstellungen zur Funktionsweise des Dialysegerätes getätigt werden können, sei es manuell, automatisch, oder durch eine Computer-gestützte Steuerung.

Der Begriff "patientenspezifische Daten" im Sinne der Erfindung bezieht sich auf Angaben, die sich auf spezifische Daten des Patienten zu Diagnose, weiteren Erkrankungen, Medikation, Anamnese und die bisherige dokumentierte Dialysebehandlung beziehen. Diese Daten sind individuell, können aber Teil eines Behandlungsprofils für ähnlich gelagerte Fälle sein.

Unter dem Begriff "Fluss" wird im Sinne der Erfindung der physikalische Fluss verstanden, der sich für Flüssigkeiten (und Gase) als das Volumen darstellt, das sich pro Zeiteinheit durch einen vorgegebenen Querschnitt eines Strömungsträgers bewegt. Oder als Formel dargestellt:

$$Q = \dot{V} = \frac{dV}{dt}$$

Hierbei steht Q für den Fluss, V für den Volumenstrom, V für das Volumen und t für die Zeit.

Der Begriff "FlusSprofile" bezieht sich erfindungsgemäß auf ein Schema der zeitlichen Abfolge von Kombinationen der Flüsse von Blut und Dialyseflüssigkeit, oder aber der Dialyseflüssigkeit alleine. Hierbei kann mindestens einer der Flüsse und/oder die zeitliche Abfolge vorgegeben sein oder sich aus der Berechnung des oder mehrerer Dialyseleistungsparameter ergeben. Bevorzugt werden die ermittelten oder vorgegebenen Flussprofile auf der zentralen Recheneinheit hinterlegt und können von dort von dem Anwender abgerufen werden. Ein derart abgelegtes Flussprofil kann als anfängliche, aber veränderbare, oder aber als definitive Grundlage des Ablaufes einer Dialysesitzung verwendet werden. Analog zu dem Begriff "Flussprofil" kann auch im Sinne der Erfindung der Begriff "Abfolgeschema der Flüsse" verwendet werden.

Der Begriff "untersten akzeptablen Werts für einen Dialyseleistungsparameter" beschreibt im Sinne der Erfindung den Grenzwert des ausgewählten Dialyseleistungsparameters, unterhalb dessen die Dialyseleistung zu keinem Zeitpunkt der Dialysesitzung abfallen soll. Dieser Grenzwert darf überschritten werden. Eine möglichst weite Überschreitung ist jedoch medizinisch nicht notwendig und oftmals ökonomisch nicht sinnvoll. Die Höhe des Grenzwerts unterscheidet sich natürlich je nach ausgewähltem Dialyseleistungsparameter. Die Auswahl und die Höhe unterliegen patientenspezifisch individuellen medizinischen Gesichtspunkten (je nach Behandlungsziel und Diagnose) und den Leistungskriterien des verwendeten Dialysegeräts und des ausgewählten Dialyseverfahrens. Es gibt keine wechselseitige Präkonditionierung zwischen der Auswahl des Dialyseleistungsparameters und seiner Höhe einerseits und der maximalen Einsparung an Dialyseflüssigkeit andererseits.

In Fig. 1 wird die Absorbanz eines UV-Messsignals in Abhängigkeit von der Dauer einer Dialysesitzung dargestellt. Es ergibt sich ein exponentieller Abfall. Dies entspricht der Beobachtung, daß am Anfang einer Dialysesitzung ein relativ hoher Messwert für die Konzentration der aus dem Blut zu entfernenden Substanz, z.B. Harnsäure, angetroffen wird. Mit zunehmender Reinigung sinkt auch die Konzentration der entfernten Substanz in der Dialyselösung und strebt asymptotisch gegen einen Minimalwert. Dieser Minimalwert ist ein Korrelat der maximalen Reinigungsleistung. Als Ausgangswert (100%) wird ein ermittelter Startwert verwendet. Zu diesem Zweck werden die ersten Messwerte ermittelt. Einen ersten Messwert kann man typischerweise nach ca. 7 min Behandlungsdauer erhalten. Aus diesen Messwerten kann man durch Extrapolation auf einen Startwert zurückrechnen. Da aber vernünftigerweise in den ersten Minuten einer Dialysesitzung noch keine Reinigung des Blutes gemessen werden kann, erfolgt die Extrapolation auf einen fiktiven Anfang der Reinigungsleistung, der einige Minuten nach Beginn der Dialysesitzung liegt. Dieser Wert wird als Ausgangswert verwendet. Aus messtechnischen Gründen läßt sich der theoretisch zu erwartende sigmoide Beginn der Kurve nicht empirisch darstellen.

In Fig. 1 sieht man anhand der Absorbanz, daß die Konzentration der absorbierenden Substanzen im Dialysat sehr schnell abfällt bzw. gegen Ende der Therapie die Kurve sehr stark abflacht, was weniger durch die Verschlechterung des Dialysatorzustandes bedingt ist, als durch eine sehr viel geringere Konzentration im Blut des Patienten.

Fig. 2 zeigt eine weitere Charakteristik der Behandlung. Dargestellt ist die Reinigungsleistung des Dialysators in Abhängigkeit des Dialysatflusses. Ohne Dialysatfluss kann keine Reinigung des Blutes erfolgen, was einer Reinigung von 0% entspricht. Erhöht man die Dialysatfluss, steigt dementsprechend die prozentuale Reinigungsleistung. Eine 100%ige Reinigungsleistung würde in dieser Darstellung bedeuten, daß 100% des in den Dialysator einfließenden Blutes gereinigt den Dialysator verlassen.

Weitere Einflussfaktoren wie Eingangskonzentration, Blutfluss und Filtereigenschaften können diesen Verlauf zusätzlich beeinflussen.

**Figurenbeschreibung**

Fig. 1:     Kurvenverlauf der Absorbanz eines UV-Messsignals in Abhängigkeit von der Behandlungsdauer

Fig. 2:     Kurvenverlauf der prozentualen Reinigungsleistung in Abhängigkeit vom Fluss der Dialyselösung

**Beispiele**

Beispiel 1:

Zur Ermittlung der folgenden Werte wird angenommen, dass nach 2 Stunden einer Dialysesitzung bei einem Dialysatfluss von 500 ml/min Dialysatfluss der Dialysatfluss reduziert werden soll, um eine unter diesen Umständen mögliche optimale Einsparung an Dialysatflüssigkeit zu erzielen. Hierzu wird der Dialysatfluss auf 400 ml/min und in einem parallelen Experiment auf 300 ml/min erniedrigt und die Dialyse so für weitere 2 Stunden laufen lassen. Dabei ergaben sich die folgenden Werte:

| BF | Kt/V(norm | Kt/V(500,400) | Kt/N(500,300) |
|---|---|---|---|
| 400 | 2.116 | 2,07 | 1,95 |
| 300 | 1,68 | 1,635 | 1,56. |
| 200 | 1,17 | 1,155 | 1,125 |
|  |  |  |  |
|  |  |  |  |
| gesp. Volume | 0 | 12 | 24 |

Hierbei bezeichnet BF den Blutfluss in ml/min; Kt/V (norm) den gemessenen Wert für den Dialyseleistungsparameter Kt/V bei einem Dialysatfluss von 500 ml/min (einem anerkannten Normwert), Kt/V (500,400) den Kt/V bei einer Reduktion des einem Dialysatflusses von 500 ml/min auf 400 ml/min und Kt/V (500,300) den Kt/V bei einer Reduktion des einem Dialysatflusses von 500 ml/min auf 300 ml/min.

In der untersten Zeile ist das eingesparte Volumen an Dialysatflüssigkeit über den Zeitraum von 2 Stunden in Litern angegeben.

Es zeigt sich, daß eine entsprechende Absenkung des Dialysatflusses nur zu einer geringen Verschlechterung des Kt/V führt. Ist der unterste akzeptable Wert für Kt/V geeignet gewählt, bzw. die Normeinstellungen für die erste Phase der Dialysesitzung mit auseichend Spielraum gewählt, bleiben die geringen Verschlechterungen von Kt/V oberhalb des untersten akzeptablen Werts für Kt/V und es kommt zu keiner Einbuße in der zuvor festgelegten Dialyseleistung für den Patienten.

Hingegen lassen sich beachtliche Mengen an Dialyseflüssigkeit bei einer derartigen erfindungsgemäßen Flusssteuerung erzielen, was ohne die erfindungsgemäße Anpassung der Flüsse nicht möglich ist (vgl. 0 Liter Einsparung ohne Anpassung).

**Patentansprüche**

1. Verfahren zur Optimierung des Verbrauchs an Dialyseflüssigkeit in einer Blutbehandlungseinheit, das die folgenden Schritte umfasst:

   a) Vorauswahl eines Operationsmodus und eines untersten akzeptablen Werts für einen Dialyseleistungsparameter;
   b) Ermittlung der aktuellen Messwerte für die Flüsse des Blutes und der Dialyseflüssigkeit in der Blutbehandlungseinheit;
   c1) Variation des Flusses der Dialyselösung in der Blutbehandlungseinheit während einer Dialysesitzung;
   c2) Messung der UV-Absorbanz mindestens einer harnpflichtigen Substanz im Dialysatausfluss oder im extrakorporalen Blutkreislauf der Blutbehandlungseinheit nach der Variation des Flusses der Dialyselösung;
   d) Weiterleitung der Messwerte der UV-Absorbanz und der aktuellen Messwerte für den Fluss des Blutes und der Dialyseflüssigkeit an eine zentrale Recheneinheit;
   e) Ermittlung, mittels der aktuellen Messwerte für den Fluss des Blutes und der Dialyseflüssigkeit und der UV-Absorptionswerte in der zentralen Recheneinheit, eines auf den Verbrauch an Dialyseflüssigkeit optimierten Flusses der Dialyselösung, und/oder des Blutes und/oder eines relativen Verhältnisses der beiden Flüsse innerhalb eines für den ausgewählten Operationsmodus festgelegten $Q_D/Q_B$-Bereichs, bei der eine Erniedrigung des Flusses der Dialyselösung keine Verringerung der Reinigungsleistung zur Folge hat, ohne daß infolgedessen der unterste akzeptable Wert des ausgewählten Dialyseleistungsparameters unterschritten wird, sodass eine optimale Sättigung der Dialyselösung an urämischen Substanzen zustande kommt;
   oder
   e') Ermittlung, mittels der aktuellen Messwerte für den Fluss des Blutes und der Dialyseflüssigkeit und der UV-Absorptionswerte in der zentralen Recheneinheit, eines auf den Verbrauch an Dialyseflüssigkeit optimierten Flusses der Dialyselösung, und/oder des Blutes und/oder eines relativen Verhältnisses des Voluminadurchsatzes der Dialyseflüssigkeit und des Blutes innerhalb eines für den ausgewählten Operationsmodus festgelegten $V_D/V_B$-Bereichs, ohne daß infolgedessen der unterste akzeptable Wert des ausgewählten Dialyseleistungsparameters unterschritten wird und eine Erniedrigung des Flusses der Dialyselösung keine Verringerung der Reinigungsleistung zur Folge hat, sodass eine optimale Sättigung der Dialyselösung an urämischen Substanzen zustande kommt;
   f) Anzeigen der ermittelten Änderung des Flusses der Dialyselösung und/oder des Blutes an der zentralen Recheneinheit und/oder automatische Nachregelung gemäß der berechneten Änderung an der Blutbehandlungseinheit; und
   g) Wiederholung der Schritte b) bis f) nach jeweils einem zuvor festgelegten Zeitintervall oder Zeitintervallschema bis zur Beendigung der Dialysesitzung.

2. ) Verfahren gemäß Anspruch 1, das die folgenden Schritte umfasst:

   a) Vorauswahl eines Operationsmodus und eines untersten akzeptablen Werts für einen Dialyseleistungsparameter;
   a') Vorauswahl eines bei der Dialysesitzung zu verbrauchenden Volumens an Dialyseflüssigkeit;
   b) Ermittlung der aktuellen Messwerte für den Fluss des Blutes und der Dialyseflüssigkeit in der Blutbehandlungseinheit;
   c) Messung der UV-Absorbanz mindestens einer harnpflichtigen Substanz im Dialysatausfluss oder im extrakorporalen Blutkreislauf der Blutbehandlungseinheit;

d) Weiterleitung der Messwerte der UV-Absorbanz und der aktuellen Messwerte für den Fluss des Blutes und der Dialyseflüssigkeit an eine zentrale Recheneinheit;

e") Ermittlung eines auf den ausgewählten Dialyseleistungsparameter optimierten Flusses oder optimierten Abfolgeschemas der Flüsse der Dialyselösung, und/oder des Blutes und/oder eines relativen Verhältnisses der beiden Flüsse innerhalb eines für den ausgewählten Operationsmodus festgelegten $Q_D/Q_B$ oder $V_D/V_B$ Bereichs in der zentralen Recheneinheit unter Wahrung des bei der Dialysesitzung zu verbrauchenden Volumens an Dialyseflüssigkeit;

f) Anzeigen der ermittelten Änderung der Flüsse der Dialyselösung und/oder des Blutes an der zentralen Recheneinheit und/oder automatische Nachregelung gemäß der berechneten Änderung an der Blutbehandlungseinheit; und

g) Wiederholung der Schritte b) bis f) nach jeweils einem zuvor festgelegten Zeitintervall oder Zeitintervallschema bis zur Beendigung der Dialysesitzung.

3. Verfahren gemäß Anspruch 1 oder 2, wobei der besagte Operationsmodus aus der Gruppe ausgewählt wird, die aus Eco Modus, Efficiency Modus und
Power Modus besteht, wobei beim Eco Modus eine der folgenden Gleichungen Anwendung findet: $0,5 < Q_D/Q_B < 1,3$, $2,0 > Q_B/Q_D > 0,77$, $0,5 < V_D/V_B < 1,3$ oder $2,0 > V_B/V_D > 0,77$, beim Efficiency Modus eine der folgenden Gleichungen Anwendung findet: $1,3 \leq Q_D/Q_B \leq 2,0$, $0,5 \leq Q_B/Q_D \leq 0,77$, $1,3 \leq V_D N_B \leq 2,0$, oder $0,5 \leq V_B/V_D \leq 0,77$ und beim Power Modus einer der folgenden Gleichungen Anwendung findet: $4,0 > Q_D/Q_B > 2,0$, $0,25 < Q_B/Q_D < 0,5$, $4,0 > V_D/V_B > 2,0$ oder $0,25 < V_B/V_D < 0,5$.

4. Verfahren gemäß einem der vorhergehenden Ansprüche, wobei der Dialyseleistungsparameter Kt/V ist.

5. Verfahren gemäß einem der vorhergehenden Ansprüche, wobei bei auffälligen photospektroskopischen Messwerten, die einen Hinweis auf einen
unerwarteten und/oder nicht ordnungsgemäßen Verlauf der Dialysesitzung
geben, die Ausgabe des Ergebnisses die Empfehlung, den Operationsmodus
zu ändern, oder die automatische Änderung des Operationsmodus umfasst.

6. Verfahren gemäß Anspruch 1, wobei in vordefinierten oder frei wählbaren Zeitintervallen der Fluss der Dialyselösung variiert wird.

7. Verfahren gemäß Anspruch 6, wobei die vordefinierten Zeitintervalle über den Verlauf der Behandlung gleich bleiben.

8. Verfahren gemäß Anspruch 6, wobei die vordefinierten Zeitintervalle zum Ende der Behandlung größer werden.

9. Verfahren gemäß Anspruch 1 oder 2, wobei bei einem auffälligen Verlauf des Signals der online-Überwachung der Fluss der Dialyselösung variiert wird.

10. Verfahren gemäß Anspruch 9, wobei der auffällige Verlauf ein sehr langsamer Abfall oder ein sehr großer Wert der entfernten Stoffmenge ist, infolgedessen eine Erhöhung des Flusses der Dialyselösung vorgeschlagen oder vorgenommen wird.

11. Verfahren gemäß Anspruch 9, wobei der auffällige Verlauf ein sehr geringer Wert der entfernten Stoffmenge ist, infolgedessen eine Erniedrigung des Flusses der Dialyselösung vorgeschlagen oder vorgenommen wird.

12. Verfahren gemäß einem der vorhergehenden Ansprüche, wobei der Verlauf der Reinigungsleistung mit einem auf der zentralen Recheneinheit hinterlegten modellhaften Verlauf verglichen wird und eine entsprechende Korrekturmaßnahme vorgeschlagen oder vorgenommen wird.

13. Verfahren gemäß einem der vorhergehenden Ansprüche, wobei der Verlauf der Reinigungsleistung durch die Aufnahme von einzelnen Messpunkten definiert und eine Kurve interpoliert wird, wobei die Messpunkte mit einer konstanten Frequenz oder auf den erwarteten Verlauf optimiert aufgenommen werden.

14. Verfahren gemäß einem der vorhergehenden Ansprüche, wobei für jeden Patienten bei seinen Dialysesitzungen ermittelten Messwerte und Flüsse auf einem Datenträger festgehalten werden und zur Wiederverwendung zur Verfügung gestellt werden.

**15.** Verfahren gemäß einem der vorhergehenden Ansprüche, wobei baubedingte Dialysatorspezifikationen zur Optimierung des Verbrauchs oder des Einsatzes an Dialyseflüssigkeit herangezogen werden.

**16.** Verfahren gemäß der Ansprüche 14 oder 15, wobei die patientenspezifischen Daten und die Dialysatorspezifikationen kombiniert werden.

**17.** Verfahren gemäß einem der Ansprüche 14 bis 16, wobei die erhobenen Daten zur Erstellung von Flussprofilen verwendet werden, die dem Anwender vorgeschlagen werden.

**18.** Verfahren gemäß einem der vorhergehenden Ansprüche, wobei der laufende Optimierungsprozess dem Anwender angezeigt wird und auf einem Datenträger gespeichert wird.

**19.** Verfahren gemäß Anspruch 18, wobei die eingesparte Menge an Dialyselösung oder die gesteigerte Dialysequalität angezeigt wird.

**20.** Vorrichtung zur Blutbehandlung, die
eine Blutbehandlungseinheit,
eine UV-Messvorrichtung und
eine zentrale Recheneinheit umfasst, wobei die zentrale Recheneinheit über Mittel verfügt, die ein Verfahren implementieren, das die folgenden Schritte umfasst:

a) Vorauswahl eines Operationsmodus und eines untersten akzeptablen Werts für einen Dialyseleistungsparameter;
b) Ermittlung der aktuellen Messwerte für die Flüsse des Blutes und der Dialyseflüssigkeit in der Blutbehandlungseinheit;
c1) Variation des Flusses der Dialyselösung in der Blutbehandlungseinheit während einer Dialysesitzung;
c2) Messung der UV-Absorbanz mindestens einer harnpflichtigen Substanz im Dialysatausfluss oder im extrakorporalen Blutkreislauf der Blutbehandlungseinheit;
d) Weiterleitung der Messwerte der UV-Absorbanz und der aktuellen Messwerte für den Fluss des Blutes und der Dialyseflüssigkeit an eine zentrale Recheneinheit;
e) Ermittlung, mittels der aktuellen Messwerte für den Fluss des Blutes und der Dialyseflüssigkeit und der UV-Absorptionswerte in der zentralen Recheneinheit, eines auf den Verbrauch an Dialyseflüssigkeit optimierten Flusses der Dialyselösung, und/oder des Blutes und/oder eines relativen Verhältnisses der beiden Flüsse innerhalb eines für den ausgewählten Operationsmodus festgelegten $Q_D/Q_B$-Bereichs, bei der eine Erniedrigung des Flusses der Dialyselösung keine Verringerung der Reinigungsleistung zur Folge hat, ohne daß infolgedessen der unterste akzeptable Wert des ausgewählten Dialyseleistungsparameters unterschritten wird, sodass eine optimale Sättigung der Dialyselösung an urämischen Substanzen zustande kommt;
oder
e') Ermittlung, mittels der aktuellen Messwerte für den Fluss des Blutes und der Dialyseflüssigkeit und der UV-Absorptionswerte in der zentralen Recheneinheit, eines auf den Verbrauch an Dialyseflüssigkeit optimierten Flusses der Dialyselösung, und/oder des Blutes und/oder eines relativen Verhältnisses des Voluminadurchsatzes der Dialyseflüssigkeit und des Blutes innerhalb eines für den ausgewählten Operationsmodus festgelegten $V_D/V_B$-Bereichs, ohne daß infolgedessen der unterste akzeptable Wert des ausgewählten Dialyseleistungsparameters unterschritten wird und eine Erniedrigung des Flusses der Dialyselösung keine Verringerung der Reinigungsleistung zur Folge hat, sodass eine optimale Sättigung der Dialyselösung an urämischen Substanzen zustande kommt;
f) Anzeigen der ermittelten Änderung des Flusses der Dialyselösung und/oder des Blutes an der zentralen Recheneinheit und/oder automatische Nachregelung gemäß der berechneten Änderung an der Blutbehandlungseinheit; und
g) Wiederholung der Schritte b) bis f) nach jeweils einem zuvor festgelegten Zeitintervall oder Zeitintervallschema bis zur Beendigung der Dialysesitzung.

**Claims**

**1.** Method for the optimization of the consumption of dialysate in a blood treatment unit comprising the following steps:

a) Preselection of an operating mode and a lowest acceptable value for a dialysis performance parameter;

b) Determining the current measured values for the flows of the blood and the dialysate in the blood treatment unit;

c1) Variation of the flow of the dialysis solution in the blood treatment unit during a dialysis session;

c2) Measuring the UV absorbance of at least one uremic substance in the dialysate outflow or in the extracorporeal blood circulation of the blood treatment unit;

d) Forwarding the measured values of the UV absorbance and the current measured values for the flow of the blood and the dialysate to a central processing unit;

e) Determining, via the current measured values for the flow of the blood and the dialysate and the values of the UV absorbance in the central processing unit, a flow of the dialysis solution optimized on the consumption of dialysate, and/or the blood, and/or a relative ratio of the two flows within a QD/QB range determined for the selected operating mode range, for which a decrease of the flow of the dialysis solution is not followed by a decrease in the clearance, without falling as a consequence below the lowest acceptable value of the selected dialysis performance parameter, such that an optimal saturation of the dialysis solution with uremic substances results;

or

e') Determining, via the current measured values for the flow of the blood and the dialysate and the values of the UV absorbance in the central processing unit, a flow of the dialysis solution optimized on the consumption of dialysate, and/or the blood, and/or a relative ratio of the volumes throughput of the dialysate and of the blood within a VD/VB range determined for the selected operating mode without falling as a consequence below the lowest acceptable value of the selected dialysis performance parameter, and a decrease of the flow of the dialysis solution is not followed by a decrease in the clearance, such that an optimal saturation of the dialysis solution with uremic substances results;

f) Displaying the determined change of the flow of the dialysis solution and/or of the blood at the central processing unit and/or automatic readjustment in accordance with the calculated change in the blood treatment unit; and

g) Repeating the steps b) to f) after a respective predetermined time interval or time interval scheme until the end of dialysis session.

2. Method according to claim 1 comprising the following steps:

a) Preselection of an operating mode and of a lowest acceptable value for a dialysis performance parameter;

a') Preselection of a volume of dialysate to be consumed during the dialysis session;

b) Determining the current measured values for the flow of the blood and the dialysate in the blood treatment unit;

c) Measuring the UV absorbance of at least one uremic substance in the dialysate outflow or in the extracorporeal blood circulation of the blood treatment unit;

d) Forwarding the measured values of the UV absorbance and the current measured values for the flow of the blood and the dialysate to a central processing unit;

e") Determining a flow optimized on the selected dialysis performance parameter or an optimized sequence scheme of the flows of the dialysis solution, and/or the blood, and/or a relative ratio of both flows within a QD/QB range or VD/VB range determined for the selected operating mode in the central processing unit in compliance with the volume of dialysate to be consumed during the dialysis session;

f) Displaying the determined change of the flows of the dialysis solution and/or of the blood at the central processing unit and/or automatic readjustment in accordance with the calculated change in the blood treatment unit; and

g) Repeating the steps b) to f) after a respective predetermined time interval or time interval scheme until the end of dialysis session.

3. Method according to claim 1 or 2, wherein said operating mode is selected from the group consisting of Eco Mode, Efficiency Mode and Power Mode, wherein one of the following equations is applied in the Eco Mode: $0,5 < QD/QB < 1.3$, $2.0 > QB/QD > 0.77$, $0.5 < VD/VB < 1.3$, or $2.0 > VB/VD > 0.77$, one of the following equations is applied in the Efficiency Mode: $1.3 \leq QD/QB \leq 2.0$, $0.5 \leq QB/QD \leq 0.77$, $1.3 \leq VD/VB \leq 2.0$, or $0.5 \leq VB/VD \leq 0.77$ one of the following equations is applied and in the Power Mode: $4.0 > QD/QB > 2.0$, $0.25 < QB/QD < 0.5$, $4.0 > VD/VB > 2.0$, or $0.25 < VB/VD < 0.5$.

4. Method according to one of the preceding claims, wherein the dialysis performance parameter is Kt/V.

5. Method according to one of the preceding claims, wherein if photospectroscopic measured values have abnormalities, which indicate evidence of an unexpected and/or incomplete process of the dialysis session, the output of the result comprises the recommendation to change the operating mode or the automatic change of the operating mode.

6. Method according to claim 1, wherein the flow of the dialysis solution is varied in predefined or freely selectable time intervals.

7. Method according to claim 6, wherein the predefined time intervals remain constant over the process of the treatment.

8. Method according to claim 6, wherein the predefined time intervals become longer toward the end of the treatment.

9. Method according to claim 1 or 2, wherein in a peculiar course of the signal of the online monitoring, the flow of the dialysis solution is varied.

10. Method according to claim 9, wherein the peculiar course is a very slow decline or a very large value of the removed substance amount, upon which as a consequence an increase of the flow of the dialysis solution is proposed or made.

11. Method according to claim 9, wherein the peculiar course is a very low value of the removed substance amount, upon which as a consequence a decrease of the flow of the dialysis solution is proposed or made.

12. Method according to one of the preceding claims, wherein the course of the clearance is compared with a model course saved on the central processing unit, and a corresponding corrective action is proposed or made.

13. Method according to one of the preceding claims, wherein the course of the clearance is defined by recording of individual measuring points and a curve is interpolated, wherein the measuring points are recorded with a constant frequency or optimized on the expected process.

14. Method according to one of the preceding claims, wherein measured values and flows determined for each patient during his dialysis sessions are recorded on a data storage medium and are made available for reuse.

15. Method according to one of the preceding claims, wherein construction-related dialyzer specifications are used for the optimization of the consumption or the use of dialysate.

16. Method according to claim 14 or 15, wherein patient-specific data and the dialyzer specifications are combined.

17. Method according to one of claims 14 to 16, wherein the collected data is used for the generation of flow profiles, which are proposed to the user.

18. Method according to one of the preceding claims, wherein the current optimization process is displayed to the user and is saved on a data storage medium.

19. Method according to claim 18, wherein the saved amount of dialysis solution or the increased dialysis quality is displayed.

20. Apparatus for the blood treatment comprising
    a blood treatment unit,
    a UV measuring device, and
    a central processing unit,
    wherein the central processing unit has means which implement a method comprising the following steps:

    a) Preselection of an operating mode and a lowest acceptable value for a dialysis performance parameter;
    b) Determining the current measured values for the flows of the blood and the dialysate in the blood treatment unit;
    c1) Variation of the flow of the dialysis solution in the blood treatment unit during a dialysis session;
    c2) Measuring the UV absorbance of at least one uremic substance in the dialysate outflow or in the extracorporeal blood circulation of the blood treatment unit after the variation of the flow of the dialysis solution;
    d) Forwarding the measured values of the UV absorbance and the current measured values for the flow of the blood and the dialysate to a central processing unit;
    e) Determining, via the current measured values for the flow of the blood and the dialysate and the values of the UV absorbance in the central processing unit, a flow of the dialysis solution optimized on the consumption of dialysate, and/or the blood, and/or a relative ratio of the two flows within a QD/QB range determined for the selected operating mode range, for which a decrease of the flow of the dialysis solution is not followed by a decrease in the clearance, without falling as a consequence below the lowest acceptable value of the selected dialysis performance parameter, such that an optimal saturation of the dialysis solution with uremic substances results;

or

e') Determining, via the current measured values for the flow of the blood and the dialysate and the values of the UV absorbance in the central processing unit, a flow of the dialysis solution optimized on the consumption of dialysate, and/or the blood, and/or a relative ratio of the volumes throughput of the dialysate and of the blood within a VD/VB range determined for the selected operating mode without falling as a consequence below the lowest acceptable value of the selected dialysis performance parameter, and a decrease of the flow of the dialysis solution is not followed by a decrease in the clearance, such that an optimal saturation of the dialysis solution with uremic substances results;

f) Displaying the determined change of the flow of the dialysis solution and/or of the blood at the central processing unit and/or automatic readjustment in accordance with the calculated change in the blood treatment unit; and

g) Repeating the steps b) to f) after a respective predetermined time interval or time interval scheme until the end of dialysis session.

## Revendications

1. Procédé d'optimisation de la consommation de liquide de dialyse dans une unité de traitement du sang, qui comprend les étapes suivantes :

a) la présélection d'un mode de fonctionnement et d'une valeur la plus basse acceptable pour un paramètre de performance de dialyse ;
b) la détermination des valeurs de mesure actuelles pour les flux de sang et du liquide de dialyse dans l'unité de traitement du sang ;
c1) la variation du flux de la solution de dialyse dans l'unité de traitement du sang pendant une séance de dialyse ;
c2) la mesure de l'absorbance d'UV d'au moins une substance contenue dans l'urine dans l'effluent de dialysat ou dans la circulation sanguine extracorporelle de l'unité de traitement du sang après la variation du flux de la solution de dialyse ;
d) la transmission des valeurs de mesures de l'absorbance d'UV et des valeurs de mesure actuelles pour le flux du sang et du liquide de dialyse à une unité de calcul centrale ;
e) la détermination, au moyen des valeurs de mesure actuelles pour le flux du sang et du liquide de dialyse et des valeurs d'absorption d'UV dans l'unité de calcul centrale, d'un flux optimisé sur la consommation de liquide de dialyse de la solution de dialyse, et/ou du sang et/ou d'un rapport relatif des deux flux dans une plage $Q_D/Q_B$ fixée pour le mode de fonctionnement choisi, dans laquelle une diminution du flux de la solution de dialyse n'entraîne pas de réduction de la performance de purification sans que, en conséquence, la valeur la plus faible acceptable du paramètre de performance de dialyse choisi soit inférieure, de sorte qu'une saturation optimale de la solution de dialyse en substances urémiques ait lieu ;
ou
e') la détermination, au moyen des valeurs de mesure actuelles pour le flux du sang et du liquide de dialyse et des valeurs d'absorption d'UV dans l'unité de calcul centrale, d'un flux optimisé sur la consommation de liquide de dialyse de la solution de dialyse, et/ou du sang et/ou d'un rapport relatif du rendement volumique du liquide de dialyse et du sang dans une plage $V_D/V_B$ fixée pour le mode de fonctionnement choisi, sans que, en conséquence, la valeur la plus faible acceptable du paramètre de performance de dialyse choisi soit inférieure et une diminution du flux de la solution de dialyse n'entraîne pas de réduction de la performance de purification de sorte qu'une saturation optimale de la solution de dialyse en substances urémiques ait lieu ;
f) l'affichage de la modification déterminée du flux de la solution de dialyse et/ou du sang sur l'unité de calcul centrale et/ou correction automatique selon la modification calculée de l'unité de traitement du sang ; et
g) la répétition des étapes b) à f) après respectivement un intervalle temporel ou un schéma d'intervalle temporel fixé précédemment jusqu'à la fin de la séance de dialyse.

2. Procédé selon la revendication 1, qui comprend les étapes suivantes :

a) la présélection d'un mode de fonctionnement et d'une valeur la plus faible acceptable pour un paramètre de performance de dialyse ;
a') la présélection d'un volume de liquide de dialyse à consommer lors de la séance de dialyse ;

b) la détermination des valeurs de mesure actuelles pour le flux du sang et du liquide de dialyse dans l'unité de traitement du sang ;

c) la mesure de l'absorbance d'UV d'au moins une substance contenue dans l'urine dans l'effluent de dialysat ou dans la circulation sanguine extracorporelle de l'unité de traitement du sang ;

d) la transmission des valeurs de mesures de l'absorbance d'UV et des valeurs de mesure actuelles pour le flux du sang et du liquide de dialyse à une unité de calcul centrale ;

e") la détermination d'un flux optimisé ou d'un schéma de séquences optimisé sur le paramètre de performance de dialyse choisi des flux de la solution de dialyse et/ou du sang et/ou d'un rapport relatif des deux flux dans une plage $Q_D/Q_B$ ou une plage $V_D/V_B$ fixé(e) pour le mode de fonctionnement choisi, dans l'unité de calcul centrale en respectant le volume à consommer de solution de dialyse lors de la séance de dialyse ;

f) l'affichage de la modification déterminée du flux de la solution de dialyse et/ou du sang sur l'unité de calcul centrale et/ou correction automatique selon la modification calculée de l'unité de traitement du sang ; et

g) la répétition des étapes b) à f) après respectivement un intervalle temporel ou un schéma d'intervalle temporel fixé précédemment jusqu'à la fin de la séance de dialyse.

3. Procédé selon la revendication 1 ou 2, dans lequel ledit mode de fonctionnement est choisi dans le groupe qui consiste en mode Eco, mode Efficience et mode Puissance, dans lequel dans le mode Eco, l'une des équations suivantes est utilisée : $0,5 < Q_D/Q_B < 1,3$, $2,0 > Q_B/Q_D > 0,77$, $0,5 < V_D/V_B < 1,3$ ou $2,0 > V_B/V_D > 0,77$, dans le mode Efficience, l'une des équations suivantes est utilisée : $1,3 \leq Q_D/Q_B \leq 2,0$, $0,5 \leq Q_B/Q_D \leq 0,77$, $1,3 \leq V_D/V_B \leq 2,0$, ou $0,5 \leq V_B/V_D \leq 0,77$ et dans le mode Puissance, l'une des équations suivantes est utilisée : $4,0 > Q_D/Q_B > 2,0$, $0,25 < Q_B/Q_D < 0,5$, $4,0 > V_D/V_B > 2,0$, ou $0,25 < V_B/V_D < 0,5$.

4. Procédé selon l'une des revendications précédentes, dans lequel le paramètre de performance de dialyse est Kt/V.

5. Procédé selon l'une des revendications précédentes, dans lequel dans le cas de valeurs photospectroscopiques qui donnent une indication sur une évolution inattendue et/ou anormale de la séance de dialyse, l'édition du résultat comprend la recommandation de modifier le mode de fonctionnement ou la modification automatique du mode de fonctionnement.

6. Procédé selon la revendication 1, dans lequel dans des intervalles temporels prédéfinis ou librement choisis, le flux de la solution de dialyse varie.

7. Procédé selon la revendication 6, dans lequel les intervalles temporels prédéfinis restent identiques au cours du traitement.

8. Procédé selon la revendication 6, dans lequel les intervalles temporels prédéfinis sont plus grands à la fin du traitement.

9. Procédé selon la revendication 1 ou 2, dans lequel lorsqu'une évolution du signal de la surveillance en ligne se produit, le flux de la solution de dialyse varie.

10. Procédé selon la revendication 9, dans lequel l'évolution qui se produit est une baisse très lente ou une valeur très grande de la quantité de substance retirée, en conséquence de quoi une augmentation du flux de la solution de dialyse est proposée ou entreprise.

11. Procédé selon la revendication 9, dans lequel l'évolution qui se produit est une valeur très faible de la quantité de substance retirée, en conséquence de quoi, une diminution du flux de la solution de dialyse est proposée ou entreprise.

12. Procédé selon l'une des revendications précédentes, dans lequel l'évolution de la performance de purification est comparée à une évolution modélisable enregistrée sur l'unité de calcul centrale et une mesure correctrice correspondante est proposée ou entreprise.

13. Procédé selon l'une des revendications précédentes, dans lequel l'évolution de la performance de purification est définie par l'enregistrement de points de mesure individuels et une courbe est interpolée, dans lequel les points de mesure sont enregistrés avec une fréquence constante ou optimisés sur l'évolution attendue.

14. Procédé selon l'une des revendications précédentes, dans lequel pour chaque patient lors de ses séances de dialyse, des valeurs de mesure déterminées et des flux sont conservées sur un support de données et sont mis à disposition pour une réutilisation.

15. Procédé selon l'une des revendications précédentes, dans lequel des spécifications du dialyseur selon la construction sont appliquées pour l'optimisation de la consommation ou de l'utilisation du liquide de dialyse.

16. Procédé selon la revendication 14 ou 15, dans lequel les données spécifiques du patient et les spécifications du dialyseur sont combinées.

17. Procédé selon l'une des revendications 14 à 16, dans lequel les données recueillies sont utilisées pour la production de profils de flux qui sont proposés à l'utilisateur.

18. Procédé selon l'une des revendications précédentes, dans lequel le processus d'optimisation en cours est indiqué à l'utilisateur et est enregistré sur un support de données.

19. Procédé selon la revendication 18, dans lequel la quantité de solution de dialyse économisée ou la qualité de dialyse augmentée est indiquée.

20. Dispositif de traitement du sang, qui comprend
une unité de traitement du sang,
un dispositif de mesure UV et
une unité de calcul centrale, dans lequel l'unité de calcul centrale dispose de moyens qui mettent en oeuvre un procédé qui comprend les étapes suivantes :

a) la présélection d'un mode de fonctionnement et d'une valeur la plus basse acceptable pour un paramètre de performance de dialyse ;
b) la détermination des valeurs de mesure actuelle pour les flux de sang et du liquide de dialyse dans l'unité de traitement du sang ;
c1) la variation du flux de la solution de dialyse dans l'unité de traitement du sang pendant une séance de dialyse ;
c2) la mesure de l'absorbance d'UV d'au moins une substance contenue dans l'urine dans l'effluent de dialysat ou dans la circulation sanguine extracorporelle de l'unité de traitement du sang ;
d) la transmission des valeurs de mesures de l'absorbance d'UV et des valeurs de mesure actuelles pour le flux du sang et du liquide de dialyse à une unité de calcul centrale ;
e) la détermination, au moyen des valeurs de mesure actuelles pour le flux du sang et du liquide de dialyse et des valeurs d'absorption d'UV dans l'unité de calcul centrale, d'un flux optimisé sur la consommation de liquide de dialyse de la solution de dialyse, et/ou du sang et/ou d'un rapport relatif des deux flux dans une plage $Q_D/Q_B$ fixée pour le mode de fonctionnement choisi, dans laquelle une diminution du flux de la solution de dialyse n'entraîne pas de réduction de la performance de purification sans que, en conséquence, la valeur la plus faible acceptable du paramètre de performance de dialyse choisi soit inférieure, de sorte qu'une saturation optimale de la solution de dialyse en substances urémiques ait lieu ;
ou
e') la détermination, au moyen des valeurs de mesure actuelles pour le flux du sang et du liquide de dialyse et des valeurs d'absorption d'UV dans l'unité de calcul centrale, d'un flux optimisé sur la consommation de liquide de dialyse de la solution de dialyse, et/ou du sang et/ou d'un rapport relatif du rendement volumique du liquide de dialyse et du sang dans une plage $V_D/V_B$ fixée pour le mode de fonctionnement choisi, sans que, en conséquence, la valeur la plus faible acceptable du paramètre de performance de dialyse choisi soit inférieure et une diminution du flux de la solution de dialyse n'entraîne pas de réduction de la performance de purification de sorte qu'une saturation optimale de la solution de dialyse en substances urémiques ait lieu ;
f) l'affichage de la modification déterminée du flux de la solution de dialyse et/ou du sang sur l'unité de calcul centrale et/ou correction automatique selon la modification calculée de l'unité de traitement du sang ; et
g) la répétition des étapes b) à f) après respectivement un intervalle temporel ou un schéma d'intervalle temporel fixé précédemment jusqu'à la fin de la séance de dialyse.

## Figur 1

## Figur 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1083948 B1
- EP 1543852 B1
- EP 2163272 A